# EUROPÄISCHE PATENTANMELDUNG

(11) **EP 4 796 020 A1**
(43) Veröffentlichungstag der Anmeldung: **26.08.2026**
(21) Anmeldenummer: 26157661.5
(22) Anmeldetag: 10.02.2026
(51) Int. Cl.: A61B 1/00, A61B 1/307

(54) **RESEKTOSKOPVORRICHTUNG UND RESEKTOSKOP**

(30) Priorität: 24.02.2025 DE 102025106810
(71) Anmelder: Karl Storz SE & Co. KG, 78532 Tuttlingen (DE)
(72) Erfinder: SCHWARZ, Peter, 78532 Tuttlingen (DE); GRAF, Christian, 78532 Tuttlingen (DE)

(57) **Zusammenfassung**

Die vorliegende Erfindung betrifft eine Resektoskopvorrichtung (10), umfassend eine Einführhülse (12), die einer Kavität (14) eines Patienten zuführbar ist und einen ovalen Querschnitt (110) aufweist, und einen Schaft (18), der sich durch die Einführhülse (12) erstreckt und der einen ersten Schaftabschnitt (130) und einen zweiten Schaftabschnitt (140) aufweist, die an unterschiedlichen Positionen entlang einer Längsachse (120) des Schafts (18) angeordnet sind, wobei zumindest in einer Anwendungskonfiguration der erste Schaftabschnitt (130) in der Einführhülse (12) angeordnet und innerhalb der Einführhülse (12) drehbar ist, und wobei der zweite Schaftabschnitt (140) in der Anwendungskonfiguration außerhalb der Einführhülse (12) angeordnet ist.

## Beschreibung

Die vorliegende Erfindung betrifft eine Resektoskopvorrichtung und ein Resektoskop.

Eine Resektion ist die chirurgische Entfernung von Gewebe eines Organs oder eines Tumors. Je nach Ziel und anatomischen Gegebenheiten können verschiedene Arten von Resektionen durchgeführt werden. Bei schwer zugänglichem Gewebe kann die operative Entfernung endoskopisch mit Hilfe eines Resektoskops erfolgen. Ein solches Resektoskop kommt beispielsweise bei der transurethralen Resektion zum Einsatz, bei der erkranktes Gewebe aus der Harnblase oder von der Prostata entfernt wird. Die Operation erfolgt endoskopisch durch die Harnröhre des Patienten, ohne dass eine Inzision notwendig ist.

Ein herkömmliches Resektoskop für die transurethrale Resektion umfasst einen Schaft, an dessen distalem Endabschnitt eine Beobachtungsoptik, eine Spülvorrichtung und ein Resektionswerkzeug angeordnet sind. Der Schaft ermöglicht den Zugang zur Harnblase durch die Harnröhre und bietet Stabilität während des Eingriffs. Der distale Endabschnitt des Schafts kann daher während des Eingriffs durch Vor- und Zurückschieben des Schafts entlang der Harnröhre sowie durch Schwenken des Schafts in der Harnblase positioniert werden. Ein proximaler Abschnitt des Schafts ist mit einer Bedienbaugruppe gekoppelt, die während des Eingriffs von einem Benutzer gehalten und entsprechend bewegt wird. Die Bedienbaugruppe umfasst zudem eine Betätigungsvorrichtung, mittels derer das Resektionswerkzeug relativ zum distalen Endabschnitt bewegt werden kann.

Eine Resektion mit einem solchen herkömmlichen Resektoskop wird in der Regel wie folgt durchgeführt. Der Benutzer greift das Resektoskop an der Bedienbaugruppe. Dann schiebt er den Schaft derart durch die Harnröhre des Patienten, dass der distale Endabschnitt des Schafts in die Harnblase hineinragt. Anschließend wird die Blase mit Hilfe der Spülvorrichtung gefüllt. Nun wird die Bildgebungsvorrichtung aktiviert und der Benutzer erhält durch endoskopische Beobachtung einen Überblick über das Innere der Harnblase. Auf diese Weise kann er die zu entfernenden Gewebestellen innerhalb der Harnblase identifizieren. Um diese zu entfernen, schiebt der Benutzer den Schaft vor und schwenkt ihn nach Bedarf, bis sich der distale Endabschnitt in der Nähe der betreffenden Gewebestelle befindet. Er richtet die Bildgebungsvorrichtung daraufhin so aus, dass die Gewebestelle scharf im Sichtfeld zu sehen ist. Zum Abtragen des Gewebes betätigt der Anwender nun die Betätigungsvorrichtung, wodurch er das Resektionswerkzeug vor- und zurückschiebt.

In einem iterativen Prozess kann dadurch das Gewebe Schicht für Schicht durch Betätigung der Betätigungsvorrichtung abgetragen werden. Um einen größeren Gewebebereich mit dem Resektionswerkzeug zu erreichen, was regelmäßig erforderlich ist, verschiebt der Benutzer den Schaft zusätzlich entlang der Harnröhre.

In der beschriebenen Weise kann die transurethrale Resektion vor allem zur Behandlung eines oberflächlichen Blasenkarzinoms zur Resektion in einer Harnblase eingesetzt werden. Die Erfinder der vorliegenden Erfindung haben erkannt, dass dies für den Benutzer schwierig durchzuführen ist und Komplikationen auftreten können. Während der Resektion muss der Benutzer das Resektoskop nach dem Stand der Technik stark schwenken und intensiv entlang der Harnröhre verschieben. Darunter kann eine Präzision des Abtragens des Gewebes leiden. Zudem können Komplikationen für den Patienten auftreten, wobei insbesondere das Vor- und Zurückschieben des Schafts häufig zu postoperativen Beschwerden des Patienten führt. Beispielsweise kann es während des Eingriffs zu nicht direkt sichtbaren Verletzungen von Muskeln und Gewebe oder auch zu starken Reizungen der Schleimhäute kommen. Diese Komplikationen können in der Folge zu Harninkontinenz führen. Wie erwähnt ist das Vor- und Zurückschieben des Schafts in der Harnröhre aber regelmäßig unbedingt erforderlich, um die Behandlung vollständig durchführen zu können.

Ferner haben die Erfinder erkannt, dass durch den Einsatz einer Einführhülse die Reibung zwischen dem Schaft und der Harnröhre reduziert werden kann. Die Einführhülse kann ähnlich wie ein Trokar einen Zugang zur Harnblase bereitstellen, dabei aber eine Komponente der Resektoskopvorrichtung sein. Dabei kommt der Schaft selbst im Wesentlichen nicht mehr direkt mit der Harnröhre in Kontakt, sondern erstreckt sich innerhalb der Einführhülse.

Die Erfinder haben jedoch festgestellt, dass der Gesamtdurchmesser aus Einführhülse und Schaft größer ist als der Durchmesser eines herkömmlichen Schafts, der die Harnröhre direkt kontaktiert, wie es bei einem herkömmlichen Resektoskop der Fall ist. Durch den größeren Durchmesser kann es zu einer stärkeren Gewebedehnung kommen, was im Vergleich zur Verwendung eines Resektoskops ohne Einführhülse zu einer erhöhten Belastung des Gewebes und potenziellen Verletzungen führen kann.

Ausgehend vom Stand der Technik liegt der Erfindung die Aufgabe zugrunde, ein Resektoskop bereitzustellen, mittels dessen für einen Patienten schonend durchführbar ist.

Die Aufgabe wird erfindungsgemäß durch eine Resektoskopvorrichtung und ein Resektoskop gelöst, wie sie hierin beschrieben und in den Ansprüchen definiert sind.

Die vorliegende Erfindung sieht vor, eine Resektoskopvorrichtung bereitzustellen. Diese umfasst eine Einführhülse, die einer Kavität eines Patienten zuführbar ist und einen ovalen Querschnitt aufweist und einen Schaft, der sich durch die Einführhülse erstreckt und der einen ersten Schaftabschnitt und einen zweiten Schaftabschnitt aufweist, die an unterschiedlichen Positionen entlang einer Längsachse des Schafts angeordnet sind. Zumindest in einer Anwendungskonfiguration ist der erste Schaftabschnitt in der Einführhülse angeordnet und innerhalb der Einführhülse drehbar. In der Anwendungskonfiguration ist der zweite Schaftabschnitt außerhalb der Einführhülse angeordnet.

Die vorliegende Erfindung sieht zudem vor, ein Resektoskop mit einer erfindungsgemäßen Resektoskopvorrichtung bereitzustellen.

Die erfindungsgemäßen Merkmale ermöglichen eine schonende Resektion für den Patienten. Der zur Verfügung stehende Bauraum wird effizient ausgenutzt, wodurch der Gesamtumfang im Vergleich zu einer Einführhülse mit kreisrundem Querschnitt reduziert werden kann. Zudem kann eine Einführhülse mit einer Querschnittsgeometrie bereitgestellt werden, die in günstiger Weise an anatomische Gegebenheiten angepasst ist, weshalb unnötige Traumata vermieden werden können. Durch die erfindungsgemäßen Merkmale werden Gewebedehnungen minimiert, was das Verletzungsrisiko weiter verringert.

Die Erfinder haben zudem festgestellt, dass ein Schaft einer Resektoskopvorrichtung üblicherweise unterschiedliche Funktionseinheiten entlang seiner Länge aufweist, die jeweils unterschiedlich viel Bauraum beanspruchen. Bei einer Einführhülse mit kreisrundem Querschnitt richtet sich der Radius stets nach dem Abschnitt mit dem größten Platzbedarf. Entlang der Längserstreckung des Schafts ist dieser große Radius jedoch nicht durchgehend erforderlich, da einige Schaftabschnitte nur einen kleineren Radius benötigen.

Durch die Verwendung einer Einführhülse mit ovalem Querschnitt kann der Bauraum also effizienter genutzt werden. Insbesondere entlang der Abschnitte des Schafts, die wenig Bauraum benötigen, wie etwa der erste Schaftabschnitt, wird ein unnötig großer Radius des Querschnitts der Einführhülse vermieden.

Zusätzlich haben die Erfinder erkannt, dass durch den ovalen Querschnitt bestimmte Funktionseinheiten gemeinsam durch die Einführhülse und den Schaft gebildet werden können. Anders ausgedrückt kann die Einführhülse weitere Funktionen übernehmen, die über eine Reduzierung der Reibung hinausgehen. Diese zusätzlichen Funktionen werden durch die spezifische Formgebung des Querschnitts erreicht, sodass einerseits der Bauraum effizienter genutzt und andererseits eine weniger komplexe Resektoskopvorrichtung ermöglicht wird. Die Schaftform und die Einführhülsenform können somit gezielt zur Erreichung bestimmter Funktionalitäten zusammenwirken.

Unter einem "Resektoskop" kann ein medizinisches Instrument zur endoskopischen Entfernung von Gewebe innerhalb eines Hohlorgans, insbesondere der Harnblase und/oder der Prostata, verstanden werden. Ein Resektoskop kann minimalinvasive Eingriffe ermöglichen, insbesondere durch kontrollierte Resektionsbewegungen und/oder endoskopische Bildgebung.

Unter einer "Resektoskopvorrichtung" soll insbesondere ein, vorzugsweise funktionsfähiger Bestandteil, insbesondere eine Unterbaugruppe und/oder eine Konstruktions- und/oder eine Funktionskomponente eines Resektoskops verstanden werden. Vorzugsweise kann die Resektoskopvorrichtung das Resektoskop zumindest teilweise, vorzugsweise zumindest zu einem Großteil und besonders bevorzugt vollständig ausbilden. Beispielsweise kann die Resektoskopvorrichtung dazu eingerichtet sein, zumindest teilweise und vorzugsweise zumindest zu einem Großteil in einen Hohlraum oder in eine insbesondere künstliche und/oder natürliche Kavität, insbesondere Körperkavität, eingeführt zu werden, und zwar insbesondere um diese zu begutachten und Teile davon zu verändern, insbesondere zu entfernen. Bei der Resektoskopvorrichtung kann es sich um eine medizinische Resektoskopvorrichtung handeln.

Unter "eingerichtet" kann im Rahmen dieser Offenbarung insbesondere speziell programmiert, ausgebildet, ausgelegt und/oder ausgestattet verstanden werden. Darunter, dass ein Bauteil zu einer bestimmten Funktion eingerichtet ist, kann im Rahmen dieser Offenbarung insbesondere verstanden werden, dass das Bauteil diese bestimmte Funktion in zumindest einem Anwendungs- und/oder Betriebszustand erfüllt und/oder ausführt.

Die Einführhülse kann als ein längliches Rohr ausgebildet sein und/oder ein solches umfassen, entlang dessen Längsachse insbesondere ein Führungskanal verlaufen kann. Insbesondere kann sie dazu eingerichtet sein, einen Zugang zu der Harnblase eines Patienten bereitzustellen. Dazu kann sie in einer Harnröhre des Patienten anordenbar und/oder in diese einführbar sein. In dieser angeordnet kann sich die Längsachse und insbesondere der Führungskanal entlang der Harnröhre erstrecken. In diesem Fall erstreckt sich die Einführhülse von einer proximalen Seite zu einer distalen Seite. In dem Führungskanal können der Schaft und/oder das Resektionswerkzeug zumindest teilweise und/oder abschnittsweise geführt sein und insbesondere geführt vorschiebbar sein.

In einigen Ausführungsformen kann der Schaft in der Einführhülse geführt sein. Im Speziellen kann der Schaft, insbesondere koaxial zur Einführhülse, mit seiner Längsachse entlang einer Längsachse der Einführhülse linear geführt sein. Die Einführhülse kann den Schaft in sich aufnehmen, insbesondere an einem Abschnitt, der innerhalb der Harnröhre anordenbar ist. Ebenso können andere Baugruppen in der Einführhülse aufgenommen sein, beispielsweise ein Resektionswerkzeug mit einer Werkzeugspitze, mittels derer Gewebe abgetragen werden kann und/oder eine Spülvorrichtung mit einem Zuleitungskanal und einem Absaugkanal. Die Einführhülse kann verliersicher an dem Schaft angebracht sein. In anderen Worten kann die Einführhülse relativ zum Schaft bewegbar und am Schaft befestigt sein. Eine intuitive Bedienbarkeit kann insbesondere dann erzielt werden, wenn der Schaft, insbesondere der erste Schaftabschnitt, relativ zu der Einführhülse einen linearen Bewegungsfreiheitsgrad und einen Rotationsfreiheitsgrad aufweist, im Speziellen eine axiale Bewegbarkeit in Kombination mit einer Drehbarkeit aber einer Ortsfestigkeit bezüglich einer radialen Position. In einigen Ausführungsformen kann eine Drehposition fest und/oder festlegbar sein, beispielsweise durch eine Formgebung des zweiten Schaftabschnitts und/oder einen Vorsprung am ersten Schaftabschnitt. Die Resektoskopvorrichtung kann dazu einen durch den Benutzer bedienbaren Arretiermechanismus umfassen, mittels dessen eine axiale Position feststellbar sein kann. Eine besonders intuitive Bedienbarkeit wird für den Fall erzielt, dass der Schaft, insbesondere der erste Schaftabschnitt, relativ zur Einführhülse linear beweglich und drehbar ist.

In anderen Worten kann die Einführhülse eine Funktion in der Art eines Trokars, im Speziellen eines verliersicher angebrachten Trokars, der aus der minimal-invasiven Chirurgie bekannt ist, aufweisen. Sie kann also trokarartig einen Zugang zu der Kavität, insbesondere der Harnblase, bereitstellen.

Die Einführhülse kann eine Längsachse aufweisen, entlang derer der Schaft verschiebbar ist und/oder sich erstreckt. Die Längsachse kann parallel zu, insbesondere koaxial mit, einer Haupterstreckungsrichtung der Einführhülse verlaufen. In einem in die Kavität zumindest teilweise zugeführten Zustand kann die Längsachse des Schafts ebenso parallel zur Haupterstreckungsrichtung der Einführhülse und/oder deren Längsachse verlaufen.

Die Einführhülse kann eine kleinere Längserstreckung als der Schaft, der erste Schaftabschnitt und/oder der zweite Schaftabschnitt aufweisen. In einigen Ausführungsformen kann die Längserstreckung der Einführhülse höchstens beispielsweise höchstens 95 %, insbesondere höchsten 80 %, bevorzugt höchstens 70 %, besonders bevorzugt höchstens 50 %, der Längserstreckung des Schafts, des ersten Schaftabschnitts und/oder des zweiten Schaftabschnitts betragen. Mit der Längserstreckung kann die Erstreckung entlang der Längsachse gemeint sein. Der Schaft kann beispielsweise zwischen 20 cm und 40 cm, insbesondere zwischen 20 cm und 30 cm, bevorzugt zwischen 20 cm und 25 cm lang sein.

Die Einführhülse kann aus Edelstahl, einem medizinischen Kunststoff, Titan, Polyetheretherketon und/oder dergleichen gefertigt sein. In manchen Ausführungsformen ist die Einführhülse für eine Einmalverwendung vorgesehen, bzw. ist ein Single-Use-Produkt. An seiner Innenfläche kann die Einführhülse eine reibungsreduzierende Beschichtung aufweisen, beispielsweise aus Teflon, Silikon und/oder dergleichen.

Eine Wanddicke der Einführhülse kann beispielsweise zwischen einschließlich 0,5 mm und 1,5 mm betragen.

Die Einführhülse kann entlang ihrer Längserstreckung im Wesentlichen homogen ausgebildet sein. Das kann bedeuten, dass der Querschnitt der Einführhülse entlang ihrer Längserstreckung im Wesentlichen nicht variiert, insbesondere entlang eines Bereichs, der zur Anordnung innerhalb des Harnleiters vorgesehen ist. Ein Proximaler Abschnitt der Einführhülse kann einen abweichenden Querschnitt aufweisen. Insbesondere ist jedoch eine Innenkontur der Einführhülse, die beispielsweise den Führungskanal definiert, homogen entlang der Längserstreckung der Einführhülse ausgebildet. Eine Außenkontur kann, wie bereits beschrieben, insbesondere proximal, abweichen.

Die ovale Form des Querschnitts kann sich auf eine Außenkontur der Einführhülse und/oder eine Innenkontur der Einführhülse beziehen. Insbesondere kann der Querschnitt ovalringförmig ausgebildet sein, bzw. die Einführhülse einen ovalen Querschnitt in Form eines Ovalrings aufweisen und/oder einen ovalen, ringförmigen Querschnitt aufweisen. Mit einem Ovalring bzw. mit "ovalringförmig" kann eine Form gemeint sein, die analog zu einem Kreisring eine ovale Außenkontur und ein ovales Loch aufweist. Entsprechend kann der Querschnitt der Einführhülse eine, insbesondere ovale, Öffnung aufweisen. Der Querschnitt kann eine langgestreckte Form aufweisen und insbesondere zwei senkrecht zueinander und senkrecht zu der Längsachse der Einführhülse liegende Achsen definieren, die unterschiedlich lang sind. Insofern kann der Querschnitt asymmetrisch ausgebildet sein. In einigen Ausführungsformen weist der Querschnitt eine elliptische Form auf bzw. weist die Einführhülse einen elliptischen Querschnitt auf. Es versteht sich, dass bei den genannten Ausgestaltungen eine Wandstärke in Umfangsrichtung und/oder in Längsrichtung der Einführhülse variieren kann.

Der Querschnitt kann durch eine erste Achse und eine zweite Achse definiert sein, die senkrecht zueinander und senkrecht zur Längsachse der Einführhülse angeordnet sind, wobei die zweite Achse länger ist als die erste Achse. Die zweite Achse kann beispielsweise um einen Faktor von bis zu 1,2, insbesondere bis zu 1,5, bevorzugt bis zu 2 länger sein. Eine Innenkontur und eine Außenkontur der Einführhülse können eine voneinander abweichende oder eine gleiche Form aufweisen, wobei jeweils die Innenkontur und die Außenkontur eine ovale, insbesondere elliptische Form, aufweisen. In einigen Ausführungsformen ist die Wanddicke der Einführhülse in Umfangsrichtung im Wesentlichen homogen ausgebildet.

Die zweite Achse kann eine Länge von beispielsweise bis zu 12 mm, insbesondere bis zu 10 mm, bevorzugt bis zu 8 mm aufweisen. Die erste Achse kann eine Länge von beispielsweise bis zu 10 mm, insbesondere bis zu 8 mm, bevorzugt bis zu 6 mm aufweisen. Grundsätzlich ist dabei eine kleinere Länge wünschenswert, um eine Belastung für den Patienten kleinzuhalten.

Unter einer Kavität kann eine Harnblase und auch die Harnröhre verstanden werden. In einigen Ausführungsformen ist die Einführhülse dazu eingerichtet, derart in der Harnröhre angeordnet zu werden, dass ein distaler Abschnitt der Einführhülse in die Harnblase hineinragt. In einigen Ausführungsformen kann die Einführhülse aber auch, insbesondere geringfügig, kürzer sein als die Harnröhre des behandelten Patienten bzw. sich in der Harnröhre angeordneten Zustand nicht ganz bis zum Blasenhals erstrecken.

Unter "distal" soll insbesondere bei einer Bedienung einem Patienten zugewandt und/oder einem Bediener und/oder Benutzer abgewandt verstanden werden. Insbesondere ist proximal das Gegenteil von distal. Unter "proximal" soll insbesondere bei einer Bedienung einem Patienten abgewandt und/oder einem Bediener und/oder Benutzer zugewandt verstanden werden.

Unter einem "länglichen Teil" soll insbesondere ein Bauteil verstanden werden, dessen Haupterstreckung zumindest um einen Faktor fünf, vorzugsweise zumindest um einen Faktor zehn und besonders bevorzugt zumindest um einen Faktor zwanzig größer ist als eine größte Erstreckung des Bauteils senkrecht zu dessen Haupterstreckung, also insbesondere einem Durchmesser des Bauteils. Unter einer "Haupterstreckung" eines Bauteils, soll insbesondere dessen längste Erstreckung entlang dessen Haupterstreckungsrichtung verstanden werden. Unter einer "Haupterstreckungsrichtung" eines Bauteils soll insbesondere eine Richtung verstanden werden, welche parallel zu einer längsten Kante eines kleinsten gedachten Quaders verläuft, welcher das Bauteil gerade noch vollständig umschließt und welche vorzugsweise durch einen geometrischen Mittelpunkt und/oder durch einen Massenmittelpunkt des Bauteils verläuft.

Der Schaft kann beispielsweise ein längliches, zylindrisches, insbesondere hohlzylindrisches Element umfassen, das Instrumente, Einrichtungen und/oder dergleichen trägt, mittels derer eine Resektion durchgeführt werden kann. Er kann die Einführung der Instrumente, Einrichtungen und/oder dergleichen in die Kavität, insbesondere die Harnblase, ermöglichen. Allgemein kann mittels des Schafts also ein Zugang zu der Kavität erreicht werden. Dabei kann der Schaft an seinem proximalen Abschnitt von einem Benutzer gehalten und/oder manipuliert werden, um die Instrumente, Einrichtungen und/oder dergleichen innerhalb der Kavität auszurichten. Durch die Manipulation kann insbesondere auch die Resektion durch den Benutzer durchgeführt werden. Der Schaft kann entsprechend starr und/oder biegesteif ausgebildet sein. Er kann eine Länge beispielsweise von 10 cm bis zu 50 cm, insbesondere von 15 cm bis zu 40 cm, bevorzugt von 20 cm bis zu 30 cm, aufweisen.

Der Schaft kann entlang seiner Haupterstreckungsrichtung bzw. entlang seiner Längserstreckung entlang der Längsachse unterschiedliche Schaftabschnitte definieren, insbesondere und/oder zumindest den ersten Schaftabschnitt und den zweiten Schaftabschnitt. Der erste Schaftabschnitt kann eine andere Außenkontur aufweisen als der zweite Schaftabschnitt. Ferner kann ein dritter Schaftabschnitt vorgesehen sein, der insbesondere ebenfalls eine abweichende Außenkontur aufweist. Der erste Schaftabschnitt und der zweite Schaftabschnitt können verschiedene Funktionseinheiten tragen. Eine Funktionseinheit kann etwa eine Bildgebungsvorrichtung, eine Betätigungsvorrichtung, eine Zuleitungsvorrichtung und/oder dergleichen umfassen. Eine Zuleitungsvorrichtung kann etwa Zuleitungen, elektrische Versorgungsleitungen, ein Kraftübertragungselement und/oder dergleichen umfassen. Die Zuleitungsvorrichtung kann entsprechend dazu vorgesehen sein, eine distal angeordnete Funktionseinheit mit einem proximalen Schaftabschnitt zu verbinden und diese zu versorgen. Der Schaft kann insbesondere in einem Abschnitt, der die Zuleitungsvorrichtung trägt, einen geringen Bauraum einnehmen. In einigen Ausführungsformen ist die Zuleitungsvorrichtung im ersten Schaftabschnitt angeordnet.

Allgemein kann ein Querschnitt des ersten Schaftabschnitts einen anderen, insbesondere kleineren Umfang, aufweisen als der zweite Schaftabschnitt. Eine Außenkontur des ersten Schaftabschnitts, insbesondere eines Querschnitts des ersten Schaftabschnitts, kann sich von einer Außenkontur des zweiten Schaftabschnitts, insbesondere eines Querschnitts des zweiten Schaftabschnitts, unterscheiden.

Der Schaft kann ein distales Endstück aufweisen, das an einem distalen Schaftabschnitt angeordnet ist. Insbesondere ist der Schaft derart der Kavität des Patienten zuführbar, dass das distale Endstück innerhalb der Kavität angeordnet ist. Das distale Endstück kann Funktionseinheiten tragen, mittels derer die Resektion durchführbar ist. Das distale Endstück kann einen distalen Endabschnitt des Schafts definieren und sich beispielsweise über bis zu 5 cm, insbesondere bis zu 4 cm, bevorzugt bis zu 3 cm, des Schafts erstrecken.

Der Schaft kann, insbesondere von einem Benutzer, unabhängig von der und relativ zur Einführhülse bewegbar sein. Für eine Resektion kann der Benutzer die Einführhülse gemeinsam mit dem Schaft und dem Resektionswerkzeug der Harnröhre zuführen. Dies kann auch nacheinander geschehen, beispielsweise, wenn die verschiedenen Baugruppen unterschiedlich lang sind. Unter "gemeinsam" kann entsprechend verstanden werden, dass das Zuführen ein einzelner Vorgang sein kann und der Benutzer die einzelnen Baugruppen nicht nacheinander greifen muss und einzeln durch die Harnröhre vorschieben muss. In einem in der Harnröhre bzw. der Kavität teilweise zugeführten Zustand kann der Benutzer den Schaft weiter vorschieben, insbesondere um die Harnblase zu erkunden und/oder eine Resektion durchzuführen.

In anderen Worten kann der Schaft dazu eingerichtet sein, nach einem Zuführen in die Kavität, insbesondere bei einem Abtragen von Gewebe, innerhalb der Einführhülse bewegt zu werden. Beim Abtragen von Gewebe wird also im Unterschied zu einer herkömmlichen Resektion nicht mehr eine Baugruppe, welche in unmittelbarem Kontakt mit der Harnröhre steht, verschoben. Stattdessen wird der Schaft relativ zur Einführhülse bewegt, während die Einführhülse relativ zur Harnröhre ruht.

Gemäß den obigen Ausführungen kann der Schaft verschiedene Stellungen aufweisen, die durch eine unterschiedliche Längsstellung des Schafts zu der Einführhülse definiert sein kann. In verschiedenen Längsstellungen können unterschiedliche Schaftabschnitte innerhalb der Einführhülse angeordnet sein. Beispielsweise kann in einer ersten Längsstellung der erste Schaftabschnitt zumindest teilweise innerhalb der Einführhülse angeordnet sein. In einer zweiten Längsstellung kann der zweite Schaftabschnitt zumindest teilweise innerhalb der Einführhülse angeordnet sein. In der zweiten Längsstellung kann die Anwendungskonfiguration definiert sein. Ferner kann es weitere Längsstellungen geben, in denen eine Anwendungskonfiguration definiert ist.

Mit einer Anwendungskonfiguration kann eine relative Anordnung von Komponenten und/oder Baugruppen der Resektoskopvorrichtung gemeint sein, in der eine Resektion durchzuführbar ist. Beispielsweise können zur Resektion notwendige Funktionseinheiten wie eine Werkzeugspitze innerhalb der Harnblase angeordnet sein und in dieser bewegbar sein, insbesondere in drei Raumrichtungen und/oder um die Längsachse des Schafts rotierbar sein. Insbesondere ist in der Anwendungskonfiguration eine Werkzeugspitze der Resektoskopvorrichtung um die Längsachse des Schafts drehbar. Die Werkzeugspitze kann durch eine Drehung des ersten Schaftabschnitts in der Einführhülse drehbar sein.

Ferner kann in der Anwendungskonfiguration der erste Schaftabschnitt innerhalb der Einführhülse linear verschiebbar sein. Das kann bedeuten, dass der Schaft unabhängig von der Einführhülse vorschiebbar ist. Entsprechend kann der Schaft innerhalb der Harnblase positioniert werden.

Unter "innerhalb der Einführhülse drehbar sein" kann verstanden werden, dass der erste Schaftabschnitt um eine Längsachse der Einführhülse, des ersten Schaftabschnitts und/oder des Schafts drehbar ist. Sie kann also in der Einführhülse rotiert werden in einem Zustand, in dem die Einführhülse in der Harnröhre angeordnet ist und/oder fixiert ist. Der erste Schaftabschnitt kann entsprechend unabhängig von der Einführhülse drehbar sein. Das kann bedeuten, dass die Einführhülse im Wesentlichen ortsfest bei einer Drehung des ersten Schaftabschnitts verbleibt.

Der zweite Schaftabschnitt kann durch eine Drehung des ersten Schaftabschnitts um die Längsachse des Schafts drehbar sein. Der erste Schaftabschnitt und der zweite Schaftabschnitt können also drehfest miteinander verbunden sein.

Unter "außerhalb der Einführhülse angeordnet sein" kann verstanden werden, dass sich eine Längsachse des zweiten Schaftabschnitts und eine Längsachse der Einführhülse nicht überschneiden. Der zweite Schaftabschnitt kann entsprechend distal oder proximal der Einführhülse angeordnet sein.

Gemäß einigen Ausführungsformen weist der zweite Schaftabschnitt in zumindest eine Richtung quer zur Längsachse des Schafts eine Erstreckung auf, die größer ist als der Durchmesser eines größten dem ovalen Querschnitt der Einführhülse einbeschreibbaren Kreises. Entsprechend ist der zweite Schaftabschnitt, wenn er innerhalb der Einführhülse angeordnet ist, nicht um zumindest 180° drehbar. Die Einführhülse und der zweite Schaftabschnitt können entsprechend gemeinsam eine Drehblockiereinheit ausbilden. Ferner folgt daraus, dass der zweite Schaftabschnitt lediglich in bestimmten Drehpositionen relativ zur Einführhülse in diese einführbar ist. Diese bestimmten Drehpositionen können als "Einführdrehpositionen" bezeichnet werden. In die oben genannte Richtung kann der Schaft einen Außendurchmesser von beispielsweise bis zu 11 mm, insbesondere bis zu 9 mm, bevorzugt bis zu 7 mm, aufweisen. Mit dem "größten dem ovalen Querschnitt der Einführhülse einbeschreibbaren Kreis" kann ein Kreis gemeint sein, der der, insbesondere ovalen, Öffnung des Querschnitts der Einführhülse einbeschreibbar ist. Entsprechend kann ein kleinster Abstand vom Mittelpunkt des Querschnitts zur Innenkontur des Querschnitts dem Radius des größten dem ovalen Querschnitt der Einführhülse einbeschreibbaren Kreises entsprechen.

In manchen Ausführungsformen weist der zweite Schaftabschnitt quer zur Längsachse des Schafts derart eine Erstreckung auf, die größer ist als der Durchmesser eines größten dem ovalen Querschnitt der Einführhülse einbeschreibbaren Kreises, dass der zweite Schaftabschnitt in keiner Drehposition relativ zur Einführhülse in die Einführhülse einführbar ist.

Zur Verfügung stehender Bauraum kann effizient ausgenutzt werden, wenn zumindest in einer Zuführungskonfiguration der zweite Schaftabschnitt in der Einführhülse angeordnet und drehfest gelagert ist. Der zweite Schaftabschnitt kann also derart ausgebildet sein, dass eine Drehung in einem in der Einführhülse angeordnetem Zustand nicht möglich ist. Entsprechend können die Einführhülse und der zweite Schaftabschnitt gemeinsam eine Arretiervorrichtung ausbilden. Die Einführhülse kann entsprechend nicht nur zur Reibungsverminderung, sondern auch nur drehfesten Lagerung des Schafts dienen. In der Zuführungskonfiguration kann die Einführhülse gemeinsam mit dem Schaft der Kavität, insbesondere dem Harnleiter zuführbar sein. Eine drehfeste Lagerung des Schafts kann dabei vorteilhaft sein, da man eine gute Kontrolle über den Schaft und an diesem befindliche Funktionseinheiten haben kann.

In einigen Ausführungsformen kann der Schaft unbeweglich mit der Einführhülse in zumindest einer Zuführungskonfiguration verbunden sein. Eine lineare Verschiebbarkeit kann in der Zuführkonfiguration verhindert sein. Der Schaft kann etwa einen lösbaren Fixiermechanismus umfassen, der dazu eingerichtet ist, die Einführhülse wahlweise zu fixieren.

Die Zuführungskonfiguration und die Anwendungskonfiguration können wahlweise durch einen Benutzer durch ein relatives Verschieben des Schafts zu der Einführhülse entlang der Längsachse des Schafts einstellbar sein. Der Benutzer kann also durch eine Manipulation des Schafts wahlweise die Zuführungskonfiguration oder die Anwendungskonfiguration herstellen. Diese beiden Konfigurationen können also vergleichsweise einfach einstellbar sein. Es ist kein aufwendiger Mechanismus notwendig, mittels dessen die entsprechenden Konfigurationen erreichbar sind. Die Resektoskopvorrichtung kann also wenig komplex und bauraumsparend konstruiert werden. Der Benutzer kann also wahlweise durch das relative, insbesondere lineare, Verschieben eine drehfeste und eine drehbare Lagerung des Schafts einstellen, bzw. kann durch das relative, insbesondere lineare, Verschieben eine drehfeste und eine drehbare Lagerung des Schafts einstellbar sein.

Die Funktionalität der Einführhülse kann erweitert werden, wenn ein Rücklauf für eine Spülflüssigkeit zwischen der Einführhülse und dem ersten Schaftabschnitt ausgebildet ist. Der Rücklauf kann in der Anwendungskonfiguration geöffnet und in der Zuführungskonfiguration geschlossen sein. Entsprechend kann der Bauraum effizient ausgenutzt werden und insgesamt ein Umfang der Einführhülse kompakt gehalten werden. Mittels des Rücklaufs kann Flüssigkeit aus der Harnblase abgesaugt werden. Beispielsweise könnte Spülflüssigkeit mittels einer Spülvorrichtung der Resektoskopvorrichtung in die Harnblase eingebracht werden. Dies ist etwa notwendig, um die Harnblase zu füllen und sie zu spannen. In diesem Zustand kann Gewebe leichter bearbeitet und entfernt werden. Ferner können bei der Resektion entstehende Gewebereste abgesaugt werden. Herkömmliche Resektoskope bieten allenfalls einen Rücklauf mit kleinem Durchmesser an, mittels dessen die Harnblase nicht effizient gespült werden kann. Wird eine kreisrunde Einführhülse verwendet, könnte der Rücklauf etwa zwischen dem ebenfalls kreisrunden Schaft und der Einführhülse angeordnet sein. Allerdings bietet auch dieser Rücklauf einen insgesamt kleinen Volumenstrom. Die Erfinder haben erkannt, dass durch die Verwendung der Einführhülse mit ovalem Querschnitt und dem Schaft mit den unterschiedlichen Schaftabschnitten Bauraum innerhalb der Einführhülse in der Anwendungskonfiguration zur Verfügung steht. Dieser kann entsprechend effizient für einen leistungsstarken Rücklauf verwendet werden, der einen großen Volumenstrom bietet. Dieser kann in oben beschriebener Weise durch das relative, insbesondere lineare, Verschieben des Schafts geöffnet werden. Dies konkret dadurch, dass der zweite Schaftabschnitt eine Außenkontur mit einem größeren Umfang aufweisen kann als der erste Schaftabschnitt. Unter einem geschlossenen Rücklauf kann ein solcher verstanden werden, dessen Eingang zu einem Großteil verdeckt ist. Der Eingang muss also nicht zwangsweise vollständig bedeckt sein.

Wie bereits beschrieben, kann der Schaft und/oder die Resektoskopvorrichtung eine Spülvorrichtung umfassen, mittels derer ein durch die Resektoskopvorrichtung, insbesondere durch ein Resektionswerkzeug der Resektoskopvorrichtung, bearbeitbarer Bereich spülbar ist. Während der Abtragung von Gewebe kann abgetragenes Gewebe weggespült werden, sodass eine gute Sicht auf das zu bearbeitende Gewebe gewährleistet werden kann. Die Spülvorrichtung kann einen Kanal umfassen, der in den Schaft eingebracht ist und/oder sich entlang der Längsachse des Schafts erstreckt. Auch andere Arten einer Spülvorrichtung sind denkbar, beispielsweise eine Spülvorrichtung, die in ein Resektionswerkzeug integriert ist. Proximal kann dadurch Spülflüssigkeit in den Kanal eingeleitet und entlang des Schafts in die Kavität geleitet werden. Die Spülvorrichtung kann an einem distalen Schaftabschnitt einen Auslauf umfassen, durch den Spülflüssigkeit aus der Spülvorrichtung austreten kann. Der distale

Der Schaft bzw. der erste Schaftabschnitt kann in der Anwendungskonfiguration um 180° drehbar sein, wenn der erste Schaftabschnitt in keine Richtung quer zur Längsachse des Schafts eine Erstreckung aufweist, die größer ist als der Durchmesser eines größten dem ovalen Querschnitt der Einführhülse einbeschreibbaren Kreises. Entsprechend kann ein in der Kavität angeordneter Schaftabschnitt flexibel und ohne Beschränkung eines Drehwinkels um die Längsachse positioniert werden.

Gemäß einigen Ausführungsformen ist, insbesondere in der Anwendungskonfiguration, der erste Schaftabschnitt in der Einführhülse linear entlang der Längsachse des Schafts geführt. Der Benutzer hat dadurch eine große Kontrolle über den Schaft und kann diesen präzise innerhalb der Harnblase positionieren. Wenn der Schaft für einen Gewebeabtrag linear bewegt wird, kann der Benutzer durch die lineare Führung die Gewebeabtragungsbewegung präzise durchführen. Zur linearen Führung kann der erste Schaftabschnitt eine Innenkontur der Einführhülse zumindest abschnittsweise kontaktieren. Entsprechend ist eine radiale Bewegbarkeit des ersten Schaftabschnitts in der Einführhülse eingeschränkt.

In manchen Ausführungsformen ist der erste Schaftabschnitt radial unbeweglich in der Einführhülse aufgenommen. Dies kann beispielsweise erreicht werden, wenn der erste Schaftabschnitt und die Einführhülse gemeinsam eine Passung bilden. Insbesondere weist der erste Schaftabschnitt dabei eine Außenkontur auf, die wenigstens abschnittsweise mit einem kreisrunden Außenquerschnitt mit einem ersten Durchmesser zusammenfällt, der derart bemessen ist, dass der erste Schaftabschnitt in einen größten dem ovalen Querschnitt der Einführhülse einbeschreibbaren Kreis eingepasst ist. Der kreisrunde Außenquerschnitt mit dem ersten Durchmesser kann dabei ein gedachter Außenquerschnitt sein. Der gedachte Außenquerschnitt kann entsprechend abschnittsweise die Außenkontur des ersten Schaftabschnitts beschreiben und/oder definieren. Der erste Schaftabschnitt kann beispielsweise abschnittsweise entlang des Umfangs zumindest einen Kreisbogen definieren, der zur Kontaktierung der Innenkontur der Einführhülse ausgelegt ist.

In manchen Ausführungsformen weist die Einführhülse einen elliptischen Querschnitt auf. Die Hauptachse der Ellipse kann dem Durchmesser des Schafts im Wesentlichen entsprechen. Der Schaft weist insbesondere einen im Wesentlichen kreisrunden Querschnitt auf.

Die Einführhülse kann also gleichzeitig gemeinsam mit dem ersten Schaftabschnitt eine lineare Führungsvorrichtung, insbesondere die Passung, definieren und den Rücklauf. Der Bauraum wird effizient ausgenutzt und eine Einführhülse mit kleinem Umfang kann bereitgestellt werden. Dies wird durch den ovalen Querschnitt der Einführhülse ermöglicht.

Ferner kann der erste Schaftabschnitt proximal zum zweiten Schaftabschnitt angeordnet sein. Entsprechend können die oben genannten Merkmale darauf bezogen sein, dass der zweite Schaftabschnitt innerhalb der Harnblase in der Anwendungskonfiguration angeordnet ist. In der Anwendungskonfiguration kann also ein Abschnitt des Schafts innerhalb der Harnblase durch eine Drehung des ersten Schaftabschnitts in der Einführhülse um die Längsachse des Schafts drehbar sein.

Gemäß einigen Ausführungsformen definiert der zweite Schaftabschnitt zumindest teilweise ein distales Endstück des Schafts. Alternativ oder zusätzlich weist das distale Endstück den zweiten Schaftabschnitt auf. In anderen Worten kann der Schaft ein distales Endstück aufweisen, das abschnittsweise den zweiten Schaftabschnitt ausbildet. Der zweite Schaftabschnitt kann beispielsweise ein distaler Endabschnitt des distalen Endstücks definieren. Das distale Endstück kann entsprechend die oben genannten Merkmale des zweiten Schaftabschnitts aufweisen. Folglich kann in der Zuführungskonfiguration das distale Endstück drehfest in der Einführhülse gelagert sein. Der Schaft erstreckt sich also in diesem Fall nicht wesentlich distal über ein distales Ende der Einführhülse hinaus. Lediglich ein Teil des distalen Endstücks könnte distal hinausragen. Dadurch kann verhindert werden, dass der Schaft versehentlich zu weit in die Harnblase beim Einführen hineingeschoben wird und die Harnblase verletzt.

Das distale Endstück kann eine größere Außenkontur aufweisen als der erste Schaftabschnitt. Entsprechend kann das distale Endstück einen größeren Bauraum zur Anordnung von Funktionseinheiten zur Verfügung stellen. Die Erfinder haben erkannt, dass insbesondere an dem distalen Endstück wesentliche Funktionseinheiten der Resektionsvorrichtung anzuordnen sind, da dieses in die Harnblase eingebracht wird. Entsprechend wird am distalen Endstück viel Bauraum benötigt. Allerdings ist proximal des distalen Endstücks, insbesondere entlang des ersten Schaftabschnitts, weniger Bauraum notwendig, da hier beispielsweise lediglich elektrische Leitungen, Versorgungsleitungen etc. für die Funktionseinheiten des distalen Endstücks verlaufen. Durch die Verwendung des ovalen Querschnitts kann dies ausgenutzt werden und insgesamt der Umfang der Einführhülse reduziert werden. Das distale Endstück kann also im Wesentlichen eine Öffnung des Querschnitts der Einführhülse ausfüllen und dadurch effizient den Bauraum ausnutzen. Dabei haben die Erfinder erkannt, dass eine Verwendung eines im Wesentlichen kreisrunden Querschnitts des distalen Endstücks nicht notwendig ist. In manchen Ausführungsformen kann, wie im Folgenden noch beschrieben wird, sogar ein länglicher Querschnitt vorteilhaft für die Funktionseinheiten verwendet werden.

Der Bauraum kann effizient ausgenutzt werden, wenn der zweite Schaftabschnitt eine Bilderfassungseinrichtung umfasst, die einen Beobachtungsbereich definiert. Die Bilderfassungseinrichtung benötigt regelmäßig anteilsmäßig den meisten Bauraum in dem Schaft. Diese kann also in dem distalen Endstück bzw. an einem Schaftabschnitt mit relativ großem zur Verfügung stehenden Bauraum vorgesehen sein. Dieser Abschnitt kann, wie bereits beschrieben, zusätzlich gemeinsam mit der Einführhülse, insbesondere in der Zuführungskonfiguration, eine drehfeste Lagerung bereitstellen. Es kann also der für die Bilderfassungseinrichtung notwendige Bauraum für eine weitere Funktionserreichung genutzt werden. Insgesamt kann die Resektoskopvorrichtung dadurch kompakt ausgebildet sein.

Mittels der Bilderfassungseinrichtung kann endoskopische Bildgebung durchgeführt werden, wenn diese am distalen Endstück des Schafts angeordnet ist. Die Bilderfassungseinrichtung kann dazu eingerichtet sein, Objekte innerhalb des Beobachtungsbereichs, insbesondere ein Inneres der Kavität, abzubilden und/oder Bilddaten zu erzeugen. Ein Objekt kann beispielsweise ein Gewebe, insbesondere einen Abschnitt der Blasenwand, umfassen. Anhand der Bilddaten kann an einer Anzeigevorrichtung eine Darstellung des Innern der Kavität erzeugt werden. Über die Anzeigevorrichtung kann der Benutzer also das Innere der Harnblase und einen Fortschritt der Resektion bzw. des Abtragens von Gewebe beobachten. Die Bilderfassungseinrichtung kann, insbesondere durch den Benutzer, innerhalb der Kavität unabhängig von dem Resektionswerkzeug positionierbar sein.

Gemäß einigen Ausführungsformen kann die Bilderfassungseinrichtung zumindest eine Bilderfassungseinheit umfasst, die durch zumindest eine Eingangsoptik und/oder zumindest einen Bildsensor zur Bilderzeugung ausgebildet ist. Die zumindest eine Eingangsoptik kann insbesondere an einer distalen Endfläche des Schafts, insbesondere des zweiten Schaftabschnitts und/oder des distalen Endstücks, angeordnet sein. Entsprechend kann die Resektoskopvorrichtung kompakt ausgebildet sein und endoskopische Bildgebung ermöglicht sein. Die Eingangsoptik kann durch die lineare Führung sowie die drehbare Lagerung des ersten Schaftabschnitts in der Einführhülse präzise innerhalb der Harnblase positioniert werden. Insbesondere kann die Eingangsoptik während der Resektion durch diese Merkmale stabil gehalten und hochqualitative Bildgebung durchgeführt werden. Der Bildsensor kann etwa als CCD-Chip und/oder CMOS-Chip ausgebildet sein. Die Bilderfassungseinheit kann eine modulare Baugruppe der Bilderfassungseinheit definieren.

Eine Entfernung der Werkzeugspitze zum Gewebe kann gut abgeschätzt werden und der Benutzer die Anatomie gut beurteilen, wenn die zumindest eine Bilderfassungseinheit zur Stereobildgebung eingerichtet ist. Die Resektion kann besonders präzise und schonend durchgeführt werden. Zudem kann sich der Benutzer besser im Raum bzw. Innern der Harnblase orientieren und verschiedene Funktionseinheiten relativ zum Gewebe zielgenauer positionieren. Stereobildgebung kann etwa durch eine Erfassung von zwei räumlich versetzten Bildern eines Objekts ermöglicht sein, wodurch eine dreidimensionale Darstellung des Objekts möglich wird. Dabei werden etwa Bilder aus unterschiedlichen Blickwinkeln aufgenommen, die anschließend zu einem räumlichen Gesamtbild kombiniert werden. Dies erlaub etwa die Erzeugung von Tiefeninformationen, die zur präzisen Vermessung von Strukturen und/oder Abständen innerhalb des aufgenommenen Bildfelds genutzt werden können.

Die Erfinder haben erkannt, dass gerade bei der Stereobildgebung die Verwendung eines asymmetrischen distalen Endstücks zur effizienten Bauraumnutzung vorteilhaft ist. Da in der Regel zwei Linsen nebeneinander angeordnet werden, kann in eine Richtung quer zur Längsachse des Schafts eine größere Erstreckung notwendig sein als in eine senkrecht dazu stehende Richtung senkrecht zur Längsachse. Durch die Verwendung eines kreisrunden Querschnitts des distalen Endstücks würde dieses also einen unnötig großen Umfang aufweisen. Entsprechend kann die ovale Form des Querschnitts der Einführhülse diesem Umstand Rechnung tragen. Durch die Verwendung der zwei unterschiedlich ausgeformten Schaftabschnitte kann dennoch eine gute Positionierbarkeit bereitgestellt und/oder der Rücklauf ausgebildet werden.

Die Bilderfassungseinheit kann entsprechend eine Stereoeingangsoptik mit zwei nebeneinander angeordneten Objektiven aufweisen, wobei eine Summe der Durchmesser der Objektive größer ist als der Durchmesser eines größten dem ovalen Querschnitt der Einführhülse einbeschreibbaren Kreises. Die Objektive können etwa eine Linse, insbesondere eine Sammellinse, und/oder eine Linsenanordnung umfassen. Sie können in der Richtung nebeneinander angeordnet sein, in der der Querschnitt des distalen Endstücks und/oder des zweiten Schaftabschnitts die größte Erstreckung aufweist. Dabei müssen sie nicht zwangsweise exakt auf der entsprechend größten Achse des Querschnitts angeordnet sein. Da die Summe der Durchmesser der Objektive größer ist als der Durchmesser des größten dem ovalen Querschnitt der Einführhülse einbeschreibbaren Kreises, ist das distale Endstück innerhalb der Einführhülse lediglich beschränkt und/oder drehfest gelagert. Zudem kann der Bauraum, wie bereits beschrieben, effizient ausgenutzt werden.

Ein großer Beobachtungsbereich kann bereitgestellt werden, wenn die Bilderfassungseinrichtung zumindest zwei Bilderfassungseinheiten umfasst, die in unterschiedliche Blickrichtungen gerichtet sind. Jede Bilderfassungseinheit kann durch eine Eingangsoptik und/oder einen Bildsensor zur Bilderzeugung, insbesondere einem CCD-Chip oder CMOS-Chip, ausgebildet sein.

Die wenigstens zwei Bilderfassungseinheiten können insbesondere eine erste Bilderfassungseinheit und eine zweite Bilderfassungseinheit umfassen, wobei die erste Bilderfassungseinheit in eine distale Blickrichtung gerichtet ist, und wobei die zweite Bilderfassungseinheit in eine proximale Blickrichtung gerichtet ist. Die Blickrichtung von zwei verschiedenen Seiten verbessert die dem Benutzer zur Verfügung stehende Abbildung durch die aus verschiedenen Perspektiven aufgenommenen Bilder weiter, und verbessert insbesondere eine Einsehbarkeit beim Setzen eines Ansatzpunktes zu Entfernung von Gewebe.

In einigen Ausführungsformen umfasst die Bilderfassungseinrichtung genau zwei, genau drei und/oder mehrere bzw. zumindest zwei Bilderfassungseinheiten, die in unterschiedliche Blickrichtungen gerichtet sind, wobei die Bilderfassungseinheiten gemeinsam einen durchgängigen Beobachtungsbereich definieren. Die Beobachtungsteilbereiche definieren gemeinsam den Beobachtungsbereich und können sich insbesondere überlappen.

Die Blickrichtung kann bezüglich des Schafts in eine proximale Blickrichtung gerichtet sein. Die Blickrichtung kann bezüglich des Schafts in eine distale Blickrichtung gerichtet sein. Die Blickrichtung kann durch einen Blickwinkel bezüglich der Längsachse des Schafts definiert werden. Ein Blickwinkel von 0° bis weniger als 90° kann als in eine distale Blickrichtung gerichtet bedeuten und ein Blickwinkel von mehr als 90° bis 180° kann als in eine proximale Blickrichtung gerichtet bedeuten.

Die Bilderfassungseinrichtung kann wenigstens drei Bilderfassungseinheiten umfassen, die in drei unterschiedliche Blickrichtungen gerichtet sind. Die Anordnung mehrerer Bilderfassungseinheiten mit unterschiedlichen Blickrichtungen verbessert eine einem Benutzer zur Verfügung stehende Abbildung eines zu entfernenden Gewebes. Die wenigstens drei Bilderfassungseinheiten können drei unterschiedliche Beobachtungsteilbereiche und insbesondere jeweils einen Beobachtungsteilbereich definieren, wobei sich die unterschiedlichen Beobachtungsteilbereiche zumindest teilweise überlappen können. Die Beobachtungsteilbereiche können also jeweils einen Abschnitt aufweisen, der innerhalb eines benachbarten Beobachtungsteilbereichs liegt. Bei überlappenden Beobachtungsteilbereichen, die von verschiedenen Blickrichtungen betrachtet werden, wird die dem Benutzer zur Verfügung stehende Abbildung eines zu entfernenden Gewebes weiter verbessert.

Die überlappenden Beobachtungsteilbereiche können gemeinsam den Beobachtungsbereich definieren. Die mehreren Bilderfassungseinheiten können in einer Längsrichtung und/oder bezüglich einer Längsachse des Schafts, insbesondere des distalen Endstücks des Schafts, versetzt angeordnet sein, insbesondere hintereinander angeordnet sein.

Die eine oder die mehreren Bilderfassungseinheit/en können jeweils durch Kameramodule, insbesondere Kameramodule mit kurzbauenden Objektiven oder sogenannten CameraCubes mit Waferlevel-Optiken gebildet sein. Die eine oder mehrere Bilderfassungseinheit/en weisen insbesondere jeweils einen Einzelsichtwinkel auf. Der Einzelsichtwinkel kann beispielsweise 60°, 65°, 70°, 75°, 80°, 85°, 90°, 95°, 100°, 105° oder 110° betragen.

Die Blickrichtung einer Bilderfassungseinheit kann eine Hauptblickrichtung der jeweiligen Bilderfassungseinheit sein. Beispielsweise kann die Blickrichtung eine Mittelachse einer Eingangsoptik der betreffenden Bilderfassungseinheit sein. Der Einzelsichtwinkel kann ein Winkel sein, der bezüglich der Blickrichtung aufgespannt ist. Insbesondere kann die Mittelachse eine Winkelhalbierende des Einzelsichtwinkels sein. Je nach Ausführung der Bilderfassungseinheiten kann diese durch jeweils zwei Einzelsichtwinkel definiert sein, die senkrecht zueinander aufgespannt sind. Diese können beispielsweise in Sichtwinkel bezüglich einer Querachse bzw. einen Sichtwinkel bezüglich einer Hochachse definieren, die jeweils senkrecht zu der Mittelachse und/oder der Blickrichtung stehen. Insbesondere in Fällen, in denen eine Bilderfassungseinheit einen rechteckigen Bildsensor umfasst, können beide zu der Bilderfassungseinheit gehörenden Sichtwinkel einer kurzen und einer langen Seite des rechteckigen Bildsensors zugeordnet sein.

Die Angabe eines Einzelsichtwinkels kann auch als größter Sichtwinkel der betreffenden Bilderfassungseinheit verstanden werden und/oder als ein Sichtwinkel, der einer Diagonalen des betreffenden Bildsensors entspricht. Eine Bilderfassungseinheit kann auch durch einen horizontalen Sichtwinkel, einen vertikalen Sichtwinkel, und einen diagonalen Sichtwinkel beschreibbar sein. Angaben, die sich auf einen Sichtwinkel beziehen, können sich auf jede dieser drei Größen beziehen und beziehen sich vorzugsweise auf einen diagonalen Sichtwinkel.

Zur Beleuchtung des Innern der Kavität, der Abbildungsbereiche und/oder des Beobachtungsbereichs kann an einem distalen Endstück des Schafts eine Beleuchtungseinrichtung angeordnet sein, die dazu eingerichtet ist, Beleuchtungslicht bereitzustellen. Eine Beleuchtung des Abbildungsbereichs der Abbildungsbereiche und/oder des Beobachtungsbereichs unterstützt die Aufnahme eines Bildes davon mit der Bilderfassungseinrichtung und erhöht somit insbesondere die Bildqualität einer dem Benutzer zur Verfügung gestellten Abbildung. Die Beleuchtungseinrichtung kann mehrere Beleuchtungseinheiten umfassen, die insbesondere in unterschiedliche Beleuchtungsrichtungen gerichtet sind. Die einzelnen Beleuchtungseinheiten können durch Leuchtdioden und/oder Laserdioden und/oder Lichtleiter gebildet sein.

Eine kompakte Resektoskopvorrichtung kann bereitgestellt werden, wenn die Bilderfassungseinrichtung integral mit der Beleuchtungseinrichtung ausgebildet ist. Insbesondere das distale Endstück kann wenig raumgreifend und unter effizienter Bauraumnutzung ausgebildet sein. Die Bilderfassungseinrichtung und die Beleuchtungseinrichtung können in einigen Ausführungsformen paarweise ausgebildet sein. Das kann bedeuten, dass jeweils eine Beleuchtungseinheit integral mit einer Bilderfassungseinheit ausgebildet ist.

Ein hoher Patientenkomfort kann erreicht und postoperative Komplikationen können reduziert werden, die Resektoskopvorrichtung ein Resektionswerkzeug umfasst, wobei der Schaft und das Resektionswerkzeug unabhängig voneinander relativ zu der Einführhülse entlang der Längsachse des Schafts bewegbar sind. Der Schaft und das Resektionswerkzeug können dazu eingerichtet sein, nach einem Zuführen in die Kavität bei einem Abtragen von Gewebe innerhalb der Einführhülse bewegt zu werden. Die Einführhülse kann insbesondere dazu eingerichtet sein, bei dem Abtragen von Gewebe atraumatisch zu ruhen. Es versteht sich, dass sich die Einführhülse geringfügig relativ zum Harnleiter bewegen könnte. Diese Bewegung kann jedoch vernachlässigbar klein sein, bzw. deutlich kleiner als eine Relativbewegung zwischen einem Schaft und der Harnröhre bei einer herkömmlichen Resektion. In anderen Worten kann die Einführhülse dazu eingerichtet sein, bei einem Positionieren des Schafts innerhalb der Kavität in Bezug auf ein sie umgebendes und insbesondere sie kontaktierendes Gewebe zumindest im Wesentlichen zu ruhen.

Das Resektionswerkzeug kann eine Werkzeugspitze umfassen. Diese und/oder das Resektionswerkzeug kann dazu eingerichtet sein, ein Gewebe mittels Schneiden und/oder mittels Vaporisation zu entfernen. Das Resektionswerkzeug, insbesondere die Werkzeugspitze, kann ein elektrisch leitfähiges Material, insbesondere ein Draht, über das ein elektrischer Strom fließen kann, aufweisen. Das Resektionswerkzeug, insbesondere die Werkzeugspitze, kann als HF-Werkzeug (Hochfrequenz-Werkzeug) ausgebildet sein. Zudem kann es eine Resektionsschlinge aufweisen und/oder schlingenförmig, hakenförmig oder kugelförmig sein.

Das Resektionswerkzeug, insbesondere die Werkzeugspitze, kann für die Gewebeabtragungsbewegung unabhängig und/oder gemeinsam mit dem Schaft bewegt werden, beides unabhängig von der Einführhülse. Die Werkzeugspitze wird dabei beispielsweise vor- und zurückbewegt, während sie abzutragendes Gewebe kontaktiert. Dieses wird entsprechend schichtweise abgetragen.

Das Resektionswerkzeug, insbesondere die Werkzeugspitze, kann insbesondere eine HF-Resektionsschlinge (Hochfrequenz-Resektionsschlinge) aufweisen. Alternativ oder zusätzlich dazu kann das Resektionswerkzeug einen Laser zum Schneiden und/oder zur Vaporisation aufweisen. Der Laser kann insbesondere zur Holmium-Laser-Enukleation oder zur KTP-Laser-Vaporisation ausgebildet oder als Continuous-Wave-(Dauerstrich)-Laser ausgeführt sein. Diese Laserverfahren unterscheiden sich in der Wellenlänge und Energie des verwendeten Laserlichts, insbesondere also in ihrer Energiewirkung im Gewebe und ihrer Eindringtiefe. Alternativ oder zusätzlich dazu kann das Resektionswerkzeug, insbesondere die Werkzeugspitze, ein monopolares oder ein bipolares HF-Werkzeug (Hochfrequenz-Werkzeug) aufweisen. Das monopolares HF-Werkzeug kann als Hakenelektrode, Kugelelektrode oder Spatelelektrode zum Schneiden und/oder zur Vaporisation ausgebildet sein. Das bipolare HF-Werkzeug kann insbesondere als eine bipolare Pinzette oder Zange zum Schneiden und/oder zur Vaporisation ausgebildet sein. Das Resektionswerkzeug kann ferner dazu eingerichtet sein, Kontaktkoagulation an einem Gewebe auszuführen. Je nach Anwendungsfall kann eine beschriebene Ausgestaltung des Resektionswerkzeugs besonders geeignet sein.

Eine sichere und präzise Abtragungsbewegung kann erreicht werden, wenn der Schaft eine Führungseinrichtung für eine Längsführung des Resektionswerkzeugs umfasst. Die Führungseinrichtung kann beispielsweise eine Führungsnut umfassen, die sich entlang der Längsachse erstreckt. Sie kann etwa an einer Oberseite des Schafts in diesen eingelassen sein. Ferner kann das Resektionswerkzeug zumindest einen Trägerarm umfassen, das proximal der Werkzeugspitze angeordnet ist. Der Trägerarm kann in der Führungseinrichtung geführt und/oder aufgenommen sein.

Eine platzsparende, kompakte Resektoskopvorrichtung kann bereitgestellt werden, wenn das Resektionswerkzeug zwei Trägerarme und eine zwischen den Trägerarmen angeordnete Werkzeugspitze, insbesondere eine HF-Resektionsschlinge, umfasst. Der Schaft kann dabei zwischen den Trägerarmen angeordnet sein. Ein Trägerarm kann dazu eingerichtet sein, die Werkzeugspitze mit einer proximalen Baugruppe, insbesondere der Bedienbaugruppe, zu verbinden. Wenn die Werkzeugspitze elektrisch betrieben ist, kann eine elektrische Versorgungsleitung, beispielsweise ein Kabel, innerhalb des Trägerarms geführt sein und/oder der Trägerarm eine derartige Versorgungsleitung ausbilden. In einigen Ausführungsformen kann der Trägerarm etwa röhrenförmig ausgebildet sein und zumindest eine weitere funktionelle Einheit und/oder weitere Zuleitungen aufnehmen. Zudem kann ein Trägerarm starr und/oder mechanisch stabil ausgebildet sein. Beispielsweise kann die Werkzeugspitze relativ zu dem Schaft nach distal verfahren werden. In einem distal verfahrenen Zustand kann das Resektionswerkzeug beispielsweise bis zu 10 cm über ein distales Ende des Schafts hinausragen. Der Trägerarm ist in diesem Fall derart starr und/oder stabil und/oder steif ausgebildet, dass die Werkzeugspitze nicht nennenswert abknickt und/oder sich nicht nennenswert biegt.

Ferner kann die Resektoskopvorrichtung eine proximale Bedienbaugruppe umfassen, die als Ganzes relativ zu der Einführhülse bewegbar ist. Die Einführhülse kann also bei einer Bewegung der proximalen Bedienbaugruppe ruhen und/oder sich lediglich minimal bewegen. Der Schaft und/oder das Resektionswerkzeug können beispielsweise proximal mit der Bedienbaugruppe gekoppelt sein. Durch ein Vor- und Zurückbewegen der Bedienbaugruppe kann der Benutzer dadurch den Schaft und das Resektionswerkzeug, insbesondere gemeinsam, relativ zu der Einführhülse bewegen und insbesondere eine Abtragungsbewegung durchführen. Die proximale Bedienbaugruppe ist also durch einen Benutzer greifbar und/oder manipulierbar, wobei der Benutzer durch eine Manipulation der proximalen Bedienbaugruppe den innerhalb der Harnblase angeordneten Schaftabschnitt, insbesondere das distale Endstück, positionieren kann.

Der Schaft und das Resektionswerkzeug können also unabhängig voneinander bewegbar sein und/oder können, insbesondere durch einen Benutzer, unabhängig voneinander bewegt werden, wobei die Bewegung relativ zur Einführhülse durchgeführt wird.

In einigen Ausführungsformen umfasst die proximale Bedienbaugruppe ferner einen Betätigungsmechanismus, mittels dessen das Resektionswerkzeug entlang der Längsachse unabhängig von dem Schaft bewegbar ist. Durch eine Betätigung des Betätigungsmechanismus kann der Benutzer das Resektionswerkzeug, insbesondere unabhängig vom Schaft und/oder der Einführhülse, vor- und zurückschieben, insbesondere um eine Abtragungsbewegung durchzuführen.

In anderen Worten können der Schaft und das Resektionswerkzeug dazu eingerichtet sein, nach einem Zuführen in die Kavität bei einem Abtragen von Gewebe innerhalb der Einführhülse bewegt zu werden. Beim Abtragen von Gewebe wird also im Unterschied zu einer herkömmlichen Resektion nicht mehr eine Baugruppe, welche in Kontakt mit der Harnröhre steht, verschoben.

Die Einführhülse kann lösbar mit der Bedienbaugruppe koppelbar sein. Beispielsweise kann die Bedienbaugruppe gemeinsam mit der Einführhülse einen Koppelmechanismus ausbilden, der dazu eingerichtet ist, die Einführhülse lösbar mit der Bedienbaugruppe zu verbinden. Bedarfsweise kann die Einführhülse dadurch freigegeben werden, beispielsweise nach einem mit dem Schaft und dem Resektionswerkzeug gemeinsamen Einbringen in die Kavität. Ebenfalls kann die Einführhülse bedarfsweise wieder an die Bedienbaugruppe mechanisch gekoppelt werden, um die Einführhülse gemeinsam mit dem Schaft und dem Resektionswerkzeug aus der Kavität zu entfernen.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine schematische Darstellung einer Resektoskopvorrichtung in einer Seitenansicht;
- Fig. 2: eine schematische Darstellung eines Resektoskops umfassend die Resektoskopvorrichtung, einer Steuereinheit und einer Anzeigeeinrichtung.
- Fig. 3: eine schematische Darstellung der Resektoskopvorrichtung in einer Anwendungskonfiguration in einer Draufsicht;
- Fig. 4: einen Querschnitt der Resektoskopvorrichtung in einer Anwendungskonfiguration im Bereich der Einführhülse;
- Fig. 5.: eine schematische Darstellung einer Einführhülse und eines größten einem ovalen Querschnitt der Einführhülse einbeschreibbaren Kreises;
- Fig. 6: eine schematische Seitenansicht der Resektoskopvorrichtung in einer Zuführungskonfiguration;
- Fig. 7: eine schematische Darstellung der Resektoskopvorrichtung in der Zuführungskonfiguration in einer Draufsicht; und
- Fig. 8: eine schematische Darstellung der Resektoskopvorrichtung in der Zuführungskonfiguration in Ansicht von distal.

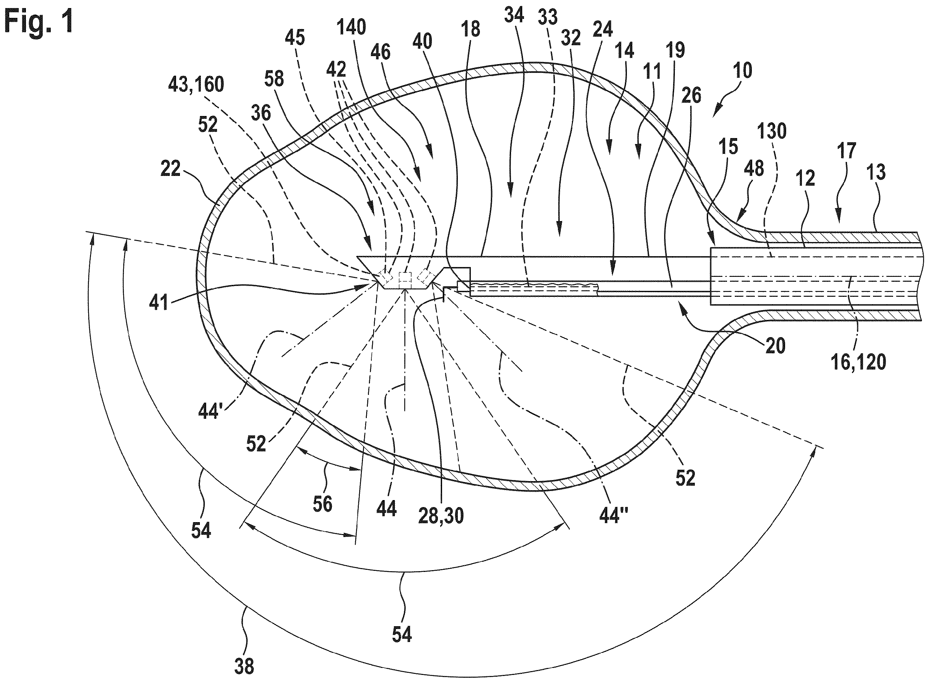

Die Fig. 1 zeigt eine schematische Darstellung einer Resektoskopvorrichtung 10 in einer Seitenansicht. Die Resektoskopvorrichtung 10 ist zur Durchführung einer Resektion eingerichtet und umfasst eine Einführhülse 12, die eine Längsachse 16 aufweist, einen Schaft 18 und ein Resektionswerkzeug 20. Die Resektoskopvorrichtung 10 ist in einer von vielen möglichen Stellungen dargestellt, konkret in einer Stellung, die eine Anwendungskonfiguration definiert. In dieser ist der Schaft 18 drehbar und linear geführt in der Einführhülse 12 gelagert. Die Stellungen werden durch eine Relativposition der Einführhülse 12, des Schafts 18 und des Resektionswerkzeugs 20 zueinander definiert. Diese Baugruppen 12, 18, 20 sind relativ zueinander unabhängig voneinander bewegbar.

Die Einführhülse 12 ist in einem in eine Harnröhre 13 eingeführten Zustand darstellt. Genauer ist ein erster Schaftabschnitt 130 in einem in der Einführhülse 12 aufgenommenen Zustand dargestellt. Ein distales Endstück 15 der Einführhülse 12 ragt in eine Harnblase 11 hinein. Denkbar ist auch, dass in anderen Situationen, beispielsweise abhängig von einer Anatomie eines Patienten, die Einführhülse 12 nicht bis in die Harnblase 11 hineinragt. Die Einführhülse 12 weist einen ovalen Querschnitt auf, wie im Folgenden noch genauer beschrieben wird. Zudem ist sie röhrenförmig ausgebildet ist und definiert einen Führungskanal 17. Sie stellt dadurch trokarartig einen Zugang zu der Harnblase 11 für den Schaft 18 und das Resektionswerkzeug 20 zur Verfügung.

Der Schaft 18 und das Resektionswerkzeug 20 erstrecken sich entlang der Längsachse 16 der Einführhülse 12 durch den Führungskanal 17 hindurch in die Kavität 14, in diesem Fall die Harnblase 11. Diese Baugruppen 18, 20 sind gemeinsam mit der Einführhülse 12 in die Harnröhre 13 einbringbar, indem sie mechanisch miteinander gekoppelt sind. Wie im Folgenden noch erläutert wird, werden der Schaft 18 und die Einführhülse vorzugsweise in einer Zuführungskonfiguration in die Harnröhre gemeinsam eingeführt. In dieser Zuführungskonfiguration ist der zweite Schaftabschnitt 140 innerhalb der Einführhülse 12 angeordnet und drehfest in ihr gelagert. Sobald die Einführhülse 12 in einer gewünschten Position angeordnet ist, können der Schaft 18 und das Resektionswerkzeug 20 weiter nach distal vorgeschoben werden. Dadurch kann die Resektoskopvorrichtung 10 von der Zuführungskonfiguration in eine Anwendungskonfiguration überführt werden. Ist der Schaft 18 derart nach distal verschoben, dass der erste Schaftabschnitt 140 nicht mehr innerhalb der Einführhülse 12 angeordnet ist, kann der Schaft 18 um seine Längsachse 120 gedreht werden.

Der Schaft 18 weist ein distales Endstück 34 auf, das unbeweglich mit einem Grundkörper 19 des Schafts 18 verbunden ist. Das distale Endstück 34 weist den zweiten Schaftabschnitt 140 auf. Der Grundkörper 19 weist den ersten Schaftabschnitt 130 auf. An dem distalen Endstück 34 weist der Schaft 18 eine Bilderfassungseinrichtung 36 für eine endoskopische Bildgebung des Innern der Harnblase 11 und eine Beleuchtungseinrichtung 46 für eine Beleuchtung des Innern der Harnblase 11 auf. Entsprechend ist die Bilderfassungseinrichtung 36 im zweiten Schaftabschnitt 140 angeordnet. Die Bilderfassungseinrichtung 36 und die Beleuchtungseinrichtung 46 sind integral miteinander ausgebildet.

Die Bilderfassungseinrichtung 36 umfasst drei Bilderfassungseinheiten 42, die unterschiedliche Blickrichtungen 44, 44', 44" definieren. Die Bilderfassungseinheiten 42 weisen darüber hinaus jeweils einen Sichtwinkel 52 auf. Eine Bilderfassungseinheit 42 weist eine Blickrichtung 44' nach distal und eine andere Bilderfassungseinheit 42 eine Blickrichtung 44" nach proximal auf. Die Bilderfassungseinheiten 42 sind jeweils durch zumindest eine Eingangsoptik 43 und/oder zumindest einen Bildsensor 45 zur Bilderzeugung ausgebildet (nur beispielhaft an einer der Bilderfassungseinheiten gezeigt). Eine der Eingangsoptiken 43 der Bilderfassungseinheiten 42 ist an einer distalen Endfläche 41 des Schafts 18, insbesondere des distalen Endstücks 34 und/oder des zweiten Schaftabschnitts 140, angeordnet. Die Bilderfassungseinheiten 42 definieren gemeinsam einen durchgängigen Beobachtungsbereich 38. Jede der Bilderfassungseinheiten 42 weist entsprechend dem jeweiligen Sichtwinkel 52 einen eigenen Beobachtungsteilbereich 54 auf. Diese weisen jeweils zumindest einen Überlappungsbereich 56 auf, der einen Beobachtungsteilbereich 54 einer benachbarten Bilderfassungseinheit teilweise überlappt. Die Bilderfassungseinheiten 42 weisen jeweils eine Stereoeingangsoptik 160 mit zwei nebeneinander angeordneten Objektiven auf (lediglich eine beispielhaft mit Bezugszeichen versehen). Dies wird im Folgenden noch genauer beschrieben. Die Bilderfassungseinheiten 42 sind integral mit Beleuchtungseinheiten (nicht im Detail dargestellt) der Beleuchtungseinrichtung 46 ausgebildet, welche als LEDs ausgebildet sind.

Das Resektionswerkzeug 20 umfasst zumindest einen Trägerarm 26 und eine Werkzeugspitze 28, die als HF-Resektionsschlinge ausgebildet ist. Genauer umfasst das Resektionswerkzeug 20 zwei Trägerarme 26. An einer Oberfläche des Schafts 18 ist eine Führungseinrichtung 24 ausgebildet, die als Führungsnut für den Trägerarm 26 ausgeführt ist. Die Führungseinrichtung 24 erstreckt sich entlang eines Großteils des Schafts 18. Das Ende der Führungseinrichtung 24 ist als offene Führungsnut vorgesehen. Die Führungseinrichtung 24, insbesondere die Führungsnut, nimmt Trägerarme 26 des Resektionswerkzeugs 20 teilweise in sich auf. Dadurch sind die Trägerarme 26 in der Führungseinrichtung 24 linear entlang einer Längsachse 120 des Schafts 18 geführt. Die Längsachse 120 ist koaxial mit der Längsachse 16 angeordnet. Der Schaft 18 und das Resektionswerkzeug 20 sind unabhängig voneinander entlang der Längsachse 120 des Schafts 18 bewegbar. Die Trägerarme 26 können also in der Führungseinrichtung 24 linear von proximal nach distal und umgekehrt verschoben werden. Durch diese Verschiebung kann die Werkzeugspitze 28 linear relativ zum Schaft 18 und unabhängig von diesem verfahren werden. Insbesondere kann die Werkzeugspitze 28 über ein distales Ende 58 des Schafts 18 hinaus verfahren werden.

Außerdem umfasst der Schaft 18 eine Spülvorrichtung 32, die einen Kanal 33 und einen Auslauf 40 umfasst. Der Kanal 33 erstreckt sich entlang der Längsachse 120 des Schafts 18 von proximal nach distal bis zu dem distalen Endstück 34 des Schafts 18. Durch den Kanal 33 ist eine Spülflüssigkeit führbar, die am Auslauf 40 austritt. Mittels der Spülflüssigkeit kann die Harnblase **11** für eine Resektion gefüllt werden und zudem Geweberückstände, die bei der Resektion anfallen, weggespült werden.

Zwischen der Einführhülse 12 und dem ersten Schaftabschnitt 130 ist zudem ein Rücklauf 48 ausgebildet, durch den Spülflüssigkeit aus der Harnblase **11** hinausbefördert werden kann. Proximal kann dazu beispielsweise eine Saugvorrichtung (nicht dargestellt) an der Einführhülse 12 angeordnet werden.

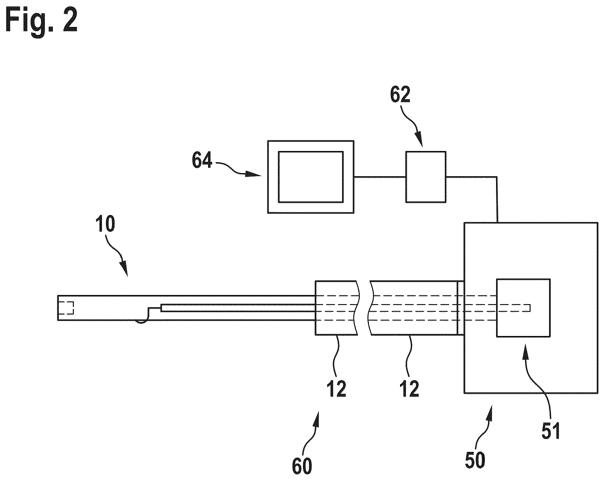

Weiterhin umfasst die Resektoskopvorrichtung 10 eine proximale Bedienbaugruppe (siehe Fig. 2), die als Ganzes relativ zu der Einführhülse 12 bewegbar ist. Ein Benutzer kann die Resektoskopvorrichtung 10 an der Bedienbaugruppe halten und ein Resektoskop umfassend die Resektoskopvorrichtung 10 (siehe Fig. 2) bedienen.

Mittels der Resektoskopvorrichtung 10 kann wie folgt eine Resektion durch den Benutzer durchgeführt werden. Zunächst führt er den Schaft 18 und das Resektionswerkzeug 20 gemeinsam mit der Einführhülse 12 der Kavität 14, vorliegend die Harnröhre 13 und teilweise die Harnblase 11, zu. Dabei bringt er die Einführhülse 12 in eine gewünschte Position innerhalb der Harnröhre 13. Nun füllt er die Harnblase 11 unter Verwendung der Spülvorrichtung 32, um diese aufzuspannen. Anschließend positioniert er das distale Endstück 34 bzw. den zweiten Schaftabschnitt 140 des Schafts 18 umfassend die Bilderfassungseinrichtung 36 innerhalb der Harnblase 11. Dazu schiebt er den Schaft 18 relativ zu der Einführhülse 12 nach distal vor. Die Einführhülse 12 verbleibt in der ursprünglichen Position.

Bei dem Vorschieben ist das Resektionswerkzeug 20 mit dem Schaft 18 gekoppelt. Das heißt, beide Baugruppen 18, 20 werden gemeinsam vorgeschoben. Durch ein Schwenken des Schafts 18 und ein bedarfsweises Vorschieben kann sich der Benutzer einen Überblick über das Innere der Harnblase 11 verschaffen und das Gewebe 22 an einer Innenwand der Harnblase 11 begutachten. Dadurch kann er abzutragendes Gewebe identifizieren, welches etwa eine pathologische Veränderung zeigt. Ein Beispiel für ein solches Gewebe ist etwa kanzeröses Gewebe.

Dieses identifizierte Gewebe kann daraufhin mittels der Werkzeugspitze 28 abgetragen werden. Dazu bringt der Benutzer das distale Endstück 34 des Schafts 18 durch Schwenken des Schafts 18 und longitudinales Positionieren des Schafts 18 in einen Nahbereich um das Gewebe. Beim longitudinalen Positionieren verbleibt die Einführhülse 12, wie bereits beschrieben, ortsfest relativ zu der Harnröhre 13 bzw. der Kavität 14. In anderen Worten ruht die Einführhülse 12 atraumatisch.

Nach dem Positionieren des distalen Endstücks 34 verfährt der Benutzer das Resektionswerkzeug 20 relativ zu dem Schaft 18, wobei die Einführhülse 12 ebenfalls ortsfest, atraumatisch ruht, und bringt die Werkzeugspitze 28 in Kontakt mit dem Gewebe. Nach Aktivierung der Werkzeugspitze 28, wenn diese etwa als HF-Resektionsschlinge ausgebildet ist, kann das Gewebe schichtweise abgetragen werden. Dazu wird die Werkzeugspitze 28 iterativ über das Gewebe gestrichen und dabei oberflächlich das abzutragende Gewebe bearbeitet und abgetragen. Zum Überstreichen des Gewebes kann einerseits das Resektionswerkzeug 20 relativ zum Schaft 18 bewegt werden. Darüber hinaus kann der Schaft 18 gemeinsam mit dem Resektionswerkzeug 20 bewegt werden, wobei jedoch die Einführhülse 12 ortsfest in der Harnröhre 13 verbleibt.

Während des Abtragens des Gewebes wird konstant mittels der Spülvorrichtung 32 gespült und Spülflüssigkeit aus der Harnblase 11 mittels des Rücklaufs 48 aus der Harnblase 11 herausbefördert.

Nach erfolgter Resektion kann der Schaft 18 gemeinsam mit dem Resektionswerkzeug 20 und der Einführhülse 12 aus der Kavität 14 herausgezogen werden.

Der Schaft 18 und das Resektionswerkzeug 20 sind also dazu eingerichtet, nach einem Zuführen in die Kavität 14 bei dem Abtragen von Gewebe 22 innerhalb der Einführhülse 12 bewegt zu werden. Genauer ist der erste Schaftabschnitt 130 während der Resektion in der Einführhülse 12 angeordnet, in dieser linear geführt aufgenommen und drehbar gelagert. Dabei ist die Einführhülse 12 dazu eingerichtet, bei dem Abtragen von Gewebe 22 atraumatisch zu ruhen.

Fig. 2 zeigt eine schematische Darstellung des Resektoskops 60, das die Resektoskopvorrichtung 10 umfasst. Das Resektoskop 60 ist stark vereinfacht dargestellt. Ferner erkennt man die proximale Bedienbaugruppe 50, die als Ganzes relativ zu der Einführhülse 12 bewegbar ist. Die proximale Bedienbaugruppe 50 umfasst zudem eine Betätigungsvorrichtung 51, mittels derer das Resektionswerkzeug 20 bedienbar, insbesondere vor- und zurückschiebbar ist.

Das Resektoskop 60 ist mit einer Steuereinheit 62 verbunden, die beispielsweise Bilddaten verarbeiten kann und eine Funktion des Resektoskops 60 steuern kann. Insbesondere kann die Bilderfassungseinrichtung mittels der Steuereinheit 62 gesteuert werden. Die Steuereinheit 62 ist mit einer Anzeigeeinrichtung 64 verbunden, an der eine Darstellung eines endoskopischen Bilds erzeugt werden kann. Über die Darstellung kann der Benutzer das Innere der Harnblase 11 betrachten und so auch die Durchführung der Resektion überwachen.

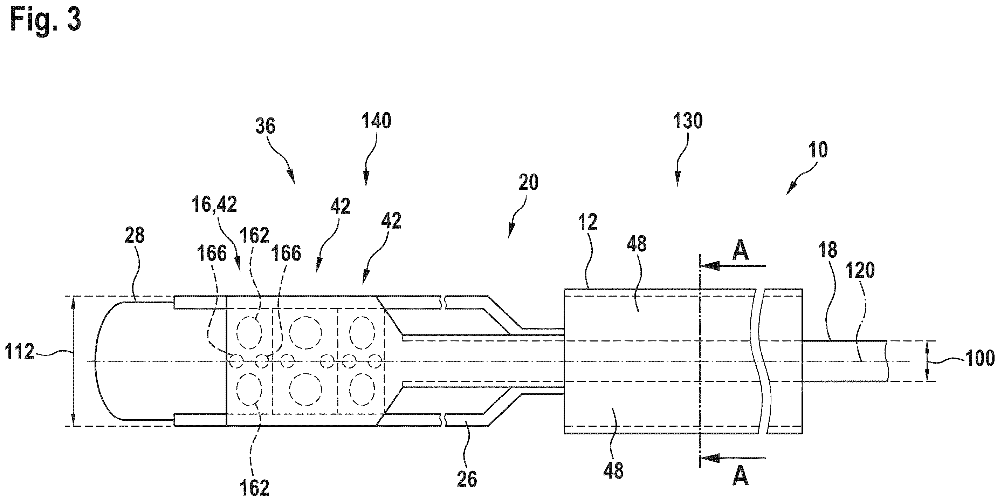

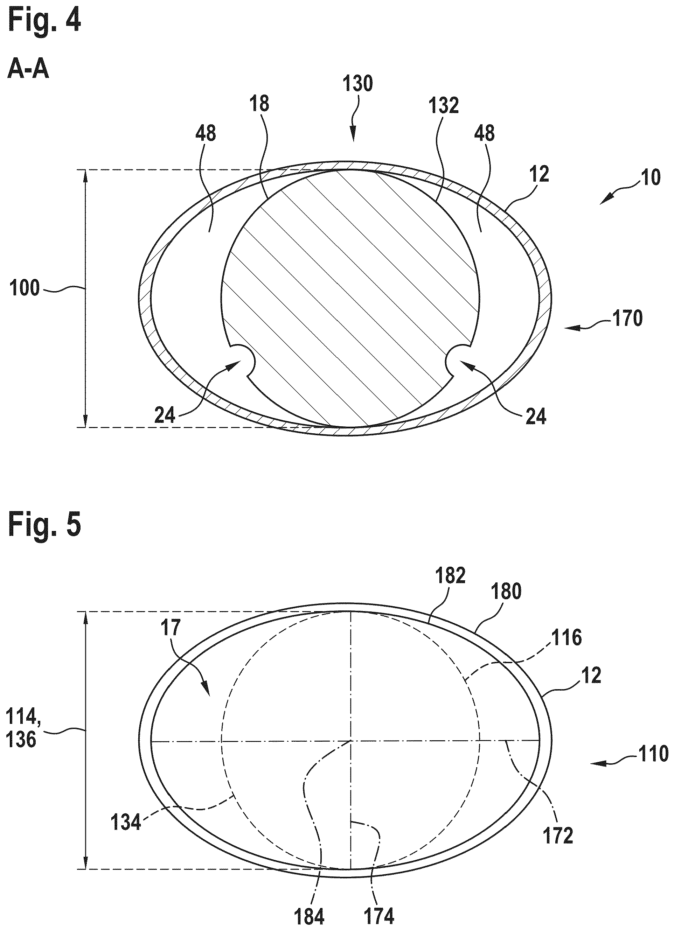

Fig. 3 und Fig. 4 zeigen die Resektoskopvorrichtung 10 in einer Anwendungskonfiguration, ähnlich der Darstellung gemäß Fig. 1. Die Trägerarme sind nicht dargestellt. Fig. 3 zeigt eine schematische Darstellung der Resektoskopvorrichtung 10 in einer Draufsicht und Fig. 4 einen Querschnitt der Resektoskopvorrichtung 10 im Bereich der Einführhülse 12. Der zweite Schaftabschnitt 140 ist distal zu dem ersten Schaftabschnitt 130 angeordnet.

Der zweite Schaftabschnitt 140 ist außerhalb der Einführhülse 12 angeordnet. Er umfasst die Bilderfassungseinrichtung 36, welche drei Bilderfassungseinheiten 42 aufweist. Diese sind im Wesentlichen baugleich ausgebildet und unterscheiden sich hinsichtlich ihres Blickwinkels (siehe Fig. 1). Man erkennt je Bilderfassungseinheit 42 zwei Objektive 162, die nebeneinander quer zur Längsachse 120 des Schafts 180 angeordnet sind. Diese bilden die Stereoeingangsoptik 160 aus. Entsprechend umfasst der zweite Schaftabschnitt 140 je Objektiv 162 einen Bilderfassungssensor (nicht dargestellt). Mit den paarweise angeordneten Objektiven 162 lässt sich also in jede Blickrichtung Stereobildgebung durchführen. Je Bilderfassungseinheit 42 sind zudem zwei LEDs 166 vorgesehen.

Der zweite Schaftabschnitt 140 weist in eine Richtung quer zur Längsachse 120 eine Erstreckung 112 auf, die größer ist als ein Schaftdurchmesser 100 des Schafts 18, insbesondere im zweiten Schaftabschnitt 130. In diese Richtung der Erstreckung sind die Objektive 162 nebeneinander angeordnet. Ferner erkennt man, dass zwischen der Einführhülse 12 und dem ersten Schaftabschnitt 130 in der Anwendungskonfiguration ein Schlitz ausgebildet ist. Dieser wird als Rücklauf 48 verwendet. Sie auch Fig. 4 für den Rücklauf 48. Dieser ist beidseitig, insbesondere in Richtung einer Hauptachse 172 der Einführhülse 12 (siehe Fig. 5) zwischen dem ersten Schaftabschnitt 130 und der Einführhülse 12 ausgebildet.

Außerdem erkennt man die Trägerarme 26, welche sich lateral entlang Längsachse 120 aufweiten. In einem proximalen Abschnitt sind sie entsprechend in dem Schaft geführt (siehe Fig. 1). Distal sind sie derart lateral aufgeweitet, dass sie lateral an den Objektiven 162 vorbeiführbar sind. Entsprechend kann die Werkzeugspitze 28 distal des Schafts 18 oder entlang des distalen Endstücks 34 angeordnet sein, je nach Relativstellung der Trägerarme 26 zum Schaft 18. Entlang des ersten Schaftabschnitts 130 sind sie jedoch nicht führbar, da die Werkzeugspitze 28 derart ausgebildet ist, dass sie gemeinsam mit dem zweiten Schaftabschnitt 140 in die Einführhülse 12 in einem Zustand einbringbar ist, in dem die Werkzeugspitze 28 in einer Längsposition entlang des zweiten Schaftabschnitts 140 angeordnet ist. Die Merkmale bzgl. des Resektionswerkzeugs 20 sind beispielhaft zu verstehen. Auch andere Ausgestaltungen eines Resektionswerkzeugs 20 sind denkbar.

Wie bereits im Zusammenhang mit Fig. 1 beschrieben, ist der Schaft 18, insbesondere der erste Schaftabschnitt 130, in der Anwendungskonfiguration drehbar in der Einführhülse 12 angeordnet. Entsprechend kann auch der zweite Schaftabschnitt 140 durch eine Drehung des ersten Schaftabschnitts 130 gedreht werden. Der erste Schaftabschnitt 130 ist also in der Einführhülse 12 angeordnet und innerhalb der Einführhülse 12 drehbar. Der zweite Schaftabschnitt 140 ist außerhalb der Einführhülse 12 angeordnet.

In der Fig. 4 erkennt man, dass der Schaft 18 im ersten Schaftabschnitt 130 einen kreisrunden Querschnitt 170 aufweist. In diesem weist er in keine Richtung quer zur Längsachse 120 des Schafts 18 eine Erstreckung auf, die größer ist als ein Durchmesser 114 eines größten einem ovalen Querschnitt 110 der Einführhülse 12 einbeschreibbaren Kreises 116. Die Einführhülse 12 sowie der Kreis 116 sind in der Fig. 5 in einer schematischen Darstellung genauer gezeigt.

In dieser erkennt man den ovalen Querschnitt 110 der Einführhülse 12. Genauer weist der Querschnitt 110 eine elliptische Ringform auf. Sie weist also eine ovale, insbesondere elliptische, Öffnung auf, die den Führungskanal 17 definiert. Ferner weist sie eine homogene Wandstärke auf, die entlang des Umfangs im Wesentlichen gleich stark ist. Der Querschnitt 110 wird entsprechend durch eine Außenkontur 180 und eine Innenkontur 182 definiert, wobei beide Konturen 180, 182 jeweils eine ovale, insbesondere elliptische, Form aufweisen, welche konzentrisch angeordnet sind. Eine Hauptachse 172 und eine Querachse 174 fallen entsprechend zusammen, wobei die Hauptachse der Außenkontur 182 entsprechend um die zweifache Wandstärke länger ist. Die Hauptachse 172 entspricht der längsten Verbindungslinie zweier auf der Innenkontur 182 angeordneten Punkt, die durch den Mittelpunkt 184 verläuft. Die Länge der Querachse 174 der elliptischen Innenkontur 182 entspricht dem Durchmesser 114 des größten dem ovalen Querschnitt 110 der Einführhülse 12 einbeschreibbaren Kreises 116. Der Durchmesser 114 entspricht also einer kürzesten Verbindungslinie zweier Punkte, die auf der Innenkontur 182 der Einführhülse 12 liegen, wobei die Verbindungslinie durch den Mittelpunkt 184 verläuft. Der Querschnitt 170 des Schafts 18 im ersten Schaftabschnitt 130 (siehe Fig. 4) entspricht im Wesentlichen dem Kreis 116.

Wie in der Fig. 4 ersichtlich, bilden der erste Schaftabschnitt 130 und die Einführhülse 12 gemeinsam eine Passung 150, da der Schaftdurchmesser 100 im Wesentlichen dem Durchmesser 114 des größten dem ovalen Querschnitt 110 der Einführhülse 12 einbeschreibbaren Kreises 116 entspricht. Der Schaftdurchmesser 100 ist geringfügig kleiner, um eine lineare Beweglichkeit des Schafts 18 in der Einführhülse 12 zu gewährleisten. Der erste Schaftabschnitt 130 weist eine Außenkontur 132 auf, die mit einem kreisrunden Außenquerschnitt 134 mit einem ersten Durchmesser 136 zusammenfällt. Der Außenquerschnitt 134 entspricht dem Kreis 116 und der erste Durchmesser 136 entsprechend dem Durchmesser 114 des Kreises 116. Der erste Durchmesser 136 ist der derart bemessen, dass der erste Schaftabschnitt 130 in den größten dem ovalen Querschnitt 110 der Einführhülse 12 einbeschreibbaren Kreis 116 eingepasst ist. Durch diese Passung ist der erste Schaftabschnitt 130 in der Anwendungskonfiguration in der Einführhülse 12 linear entlang der Längsachse 120 des Schafts 18 geführt. Eine radiale Position des ersten Schaftabschnitts 130 ist in der Einführhülse 12 definiert, bzw. der erste Schaftabschnitt 130 in der Einführhülse 12 im Wesentlichen radial unbeweglich gelagert. Eine Drehung um die Längsachse 120 bzw. um den Mittelpunkt 184 ist möglich.

Außerdem ist in der Fig. 4 die Führungseinrichtung 24 ersichtlich, welche durch zwei Führungsnuten in den ersten Schaftabschnitt 130 ausgebildet ist.

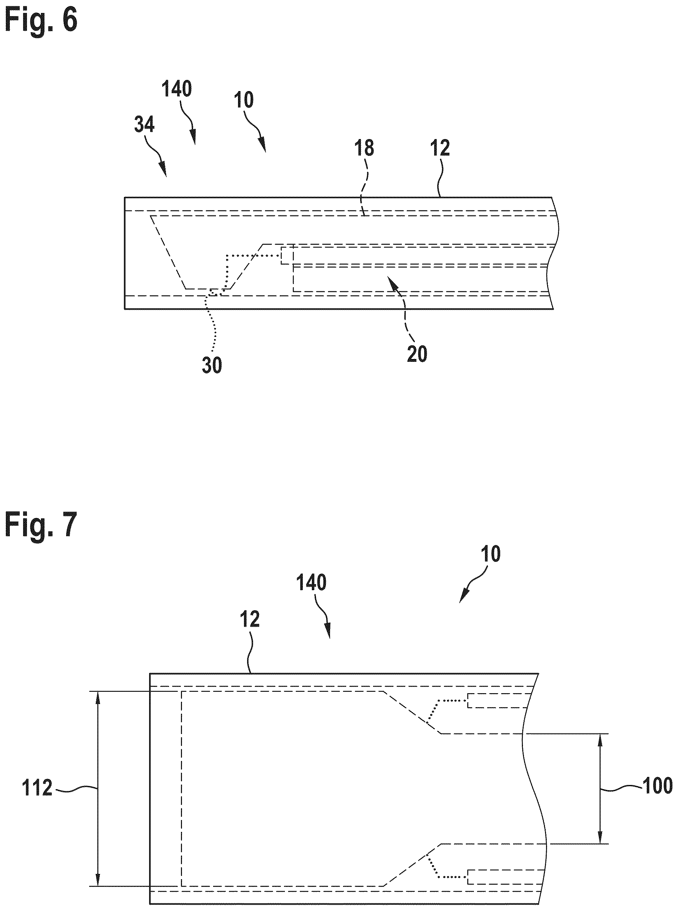

Fig. 6 zeigt eine schematische Seitenansicht der Resektoskopvorrichtung 10 in einer weiteren Stellung. Diese Stellung definiert eine Zuführungskonfiguration. In der Zuführungskonfiguration ist das distale Endstück 34 und der zweite Schaftabschnitt 140 des Schafts 18 vollständig von der Einführhülse 12 eingefasst. Das Resektionswerkzeug 20 ragt ebenfalls nicht distal über die Einführhülse 12 hinaus. In der gezeigten Stellung könnte die Resektoskopvorrichtung 10 beispielsweise der Kavität 14 zugeführt werden. Ebenso denkbar sind allerdings auch andere Stellungen, in denen zumindest eine der Baugruppen 18, 20 distale über die Einführhülse 12 hinausragt. Die HF-Resektionsschlinge 30 ist um eine Unterseite des zweiten Schaftabschnitts 140 gelegt.

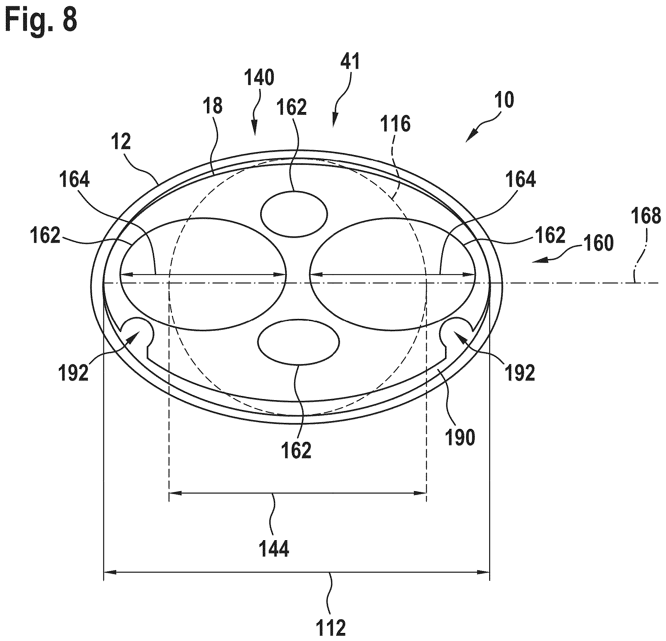

Fig. 7 und Fig. 8 zeigen die Resektoskopvorrichtung 10 in der Zuführungskonfiguration, ähnlich der Darstellung gemäß Fig. 6. Die Trägerarme sind nicht dargestellt. Fig. 7 zeigt eine schematische Darstellung der Resektoskopvorrichtung 10 in einer Draufsicht und Fig. 8 eine Ansicht von distal auf die Resektoskopvorrichtung 10. Der zweite Schaftabschnitt 140 ist in der Einführhülse 12 angeordnet und drehfest gelagert. Die drehfeste Lagerung wird durch die Formgebung des zweiten Schaftabschnitts 140 erreicht. Dieser weist die Erstreckung 112 auf (siehe auch Fig. 3), die größer ist als der Durchmesser 114. Der Kreis 116 und der Durchmesser 114 sind in zur Verdeutlichung nochmals in der Fig. 8 dargestellt. Zur Erläuterung wird auf Fig. 5 und Fig. 4 und zugehörige Beschreibung verwiesen. Die Erstreckung 112 ist in Richtung der Hauptachse 172 und quer zur Längsachse 120 des Schafts 18 angeordnet. Die Erstreckung 112 ist entsprechend größer als der Schaftdurchmesser 100. Folglich ist die Erstreckung größer als der Durchmesser 114 des größten dem ovalen Querschnitt 110 der Einführhülse 12 einbeschreibbaren Kreises 116, welcher, wie beschrieben, im Wesentlichen dem Schaftdurchmesser 100 entspricht. Wie in der Fig. 8 ersichtlich, ist der zweite Schaftabschnitt 140 passgenau in die Einführhülse 12 eingepasst. Da die Erstreckung 112 im Wesentlichen der Länge der Hauptachse 172 (siehe Fig. 5) entspricht, ist eine Drehung des Schafts 18 blockiert. Zudem ist der Rücklauf (siehe Fig. 3 und 4) geschlossen. Ferner erkennt man einen Spalt 190, durch den beispielsweise die HF-Resektionsschlinge geführt werden kann. Zur Führung der Trägerarme sind die Nuten 192 vorgesehen. Entsprechend passen die Trägerarme gemeinsam mit dem zweiten Schaftabschnitt 140 und der HF-Resektionsschlinge in die Einführhülse 12.

Außerdem ist die Stereoeingangsoptik 160 ersichtlich. Die Objektive 162 sind kreisrund ausgebildet. Da die Endfläche 41 schräg bzgl. der Längsachse 120 angeordnet ist (siehe Fig. 1), sind die Objektive 162 oval dargestellt. Zudem sind die zwei LEDs 166 zu sehen. Die Objektive 162 sind in Richtung der Hauptachse 172 (siehe Fig. 5) nebeneinander angeordnet, entsprechend in Richtung der größten Erstreckung des zweiten Schaftabschnitts 140. Die Objektive 162 sind als Linsen ausgebildet und weisen jeweils einen Durchmesser 164 auf. Eine Summe der Durchmesser 164 der Objektive 162 ist größer ist als der Durchmesser 114 des größten dem ovalen Querschnitt 110 der Einführhülse 12 einbeschreibbaren Kreises 116 (siehe Fig. 5). Die Summe ist entsprechend größer als der Schaftdurchmesser 100.

Wie bereits beschrieben sind die Zuführungskonfiguration und die Anwendungskonfiguration wahlweise durch den Benutzer durch das relative, lineare Verschieben des Schafts 18 zu der Einführhülse 12 entlang der Längsachse 120 des Schafts 18 einstellbar. Möchte er während einer Resektion den Schaft 18 etwa drehsicher lagern, um etwa sich kurzzeitig einer anderen Tätigkeit zu widmen, kann er den Schaft 18 derart um seine Längsachse 120 drehen, dass eine Haupterstreckungsachse 168 des zweiten Schaftabschnitts 140, in Richtung welcher der zweite Schaftabschnitt 140 seine größte Erstreckung aufweist, parallel zu der Hauptachse 172 ausgerichtet ist. In dieser relativen Winkelstellung des Schafts 18 zu der Einführhülse 12 ist der zweite Schaftabschnitt 140 in die Einführhülse 12 einführbar.

### Bezugszeichenliste

- 10: Resektoskopvorrichtung
- 11: Harnblase
- 12: Einführhülse
- 13: Harnröhre
- 14: Kavität
- 15: distales Endstück
- 16: Längsachse
- 17: Führungskanal
- 18: Schaft
- 19: Grundkörper
- 20: Resektionswerkzeug
- 22: Gewebe
- 24: Führungseinrichtung
- 26: Trägerarm
- 27: weiterer Trägerarm
- 28: Werkzeugspitze
- 30: HF-Resektionsschlinge
- 32: Spülvorrichtung
- 33: Zuleitung
- 34: distales Endstück
- 36: Bilderfassungseinrichtung
- 38: Beobachtungsbereich
- 40: Auslauf
- 41: distale Endfläche
- 42: Bilderfassungseinheit
- 43: Eingangsoptik
- 44: Blickrichtung
- 45: Bildsensor
- 46: Beleuchtungseinrichtung
- 47: Beleuchtungseinheit
- 48: Rücklauf
- 49: Spalt
- 50: proximale Bedienbaugruppe
- 51: Betätigungsvorrichtung
- 52: Sichtwinkel
- 54: Beobachtungsteilbereich
- 56: Überlappungsbereich
- 58: distales Ende
- 59: distale Endfläche
- 60: Resektoskop
- 62: Steuereinheit
- 64: Anzeigeeinrichtung
- 100: Schaftdurchmesser
- 110: Querschnitt
- 112: Erstreckung
- 114: Durchmesser
- 116: Kreis
- 120: Längsachse
- 130: erster Schaftabschnitt
- 132: Außenkontur
- 134: Außenquerschnitt
- 136: erster Durchmesser
- 140: zweiter Schaftabschnitt
- 150: Passung
- 160: Stereoeingangsoptik
- 162: Objektiv
- 164: Durchmesser
- 166: LED
- 168: Haupterstreckungsachse
- 170: Querschnitt
- 172: Hauptachse
- 174: Querachse
- 180: Außenkontur
- 182: Innenkontur
- 184: Mittelpunkt
- 190: Spalt
- 192: Nut

## Patentansprüche

1. Resektoskopvorrichtung (10), umfassend:
eine Einführhülse (12), die einer Kavität (14) eines Patienten zuführbar ist und einen ovalen Querschnitt (110) aufweist; und
einen Schaft (18), der sich durch die Einführhülse (12) erstreckt und der einen ersten Schaftabschnitt (130) und einen zweiten Schaftabschnitt (140) aufweist, die an unterschiedlichen Positionen entlang einer Längsachse (120) des Schafts (18) angeordnet sind,
wobei zumindest in einer Anwendungskonfiguration der erste Schaftabschnitt (130) in der Einführhülse (12) angeordnet und innerhalb der Einführhülse (12) drehbar ist, und wobei der zweite Schaftabschnitt (140) in der Anwendungskonfiguration außerhalb der Einführhülse (12) angeordnet ist.

2. Resektoskopvorrichtung (10) nach Anspruch 1,
wobei der zweite Schaftabschnitt (140) in zumindest eine Richtung quer zur Längsachse (120) des Schafts (18) eine Erstreckung (112) aufweist, die größer ist als der Durchmesser (114) eines größten dem ovalen Querschnitt (110) der Einführhülse (12) einbeschreibbaren Kreises (116).

3. Resektoskopvorrichtung (10) nach Anspruch 1 oder 2,
wobei zumindest in einer Zuführungskonfiguration der zweite Schaftabschnitt (140) in der Einführhülse (12) angeordnet und drehfest gelagert ist, und wobei insbesondere die Zuführungskonfiguration und die Anwendungskonfiguration wahlweise durch einen Benutzer durch ein relatives Verschieben des Schafts (18) zu der Einführhülse (12) entlang der Längsachse (120) des Schafts (18) einstellbar sind.

4. Resektoskopvorrichtung (10) nach Anspruch 3,
wobei ein Rücklauf (48) für eine Spülflüssigkeit zwischen der Einführhülse (12) und dem ersten Schaftabschnitt (130) ausgebildet ist, und
wobei der Rücklauf (48) in der Anwendungskonfiguration geöffnet und in der Zuführungskonfiguration geschlossen ist.

5. Resektoskopvorrichtung (10) nach einem der vorhergehenden Ansprüche,
wobei der erste Schaftabschnitt (130) in keine Richtung quer zur Längsachse (120) des Schafts (18) eine Erstreckung aufweist, die größer ist als der Durchmesser (114) eines größten dem ovalen Querschnitt der Einführhülse (12) einbeschreibbaren Kreises (116).

6. Resektoskopvorrichtung (10) nach einem der vorhergehenden Ansprüche,
wobei, insbesondere in der Anwendungskonfiguration, der erste Schaftabschnitt (130) in der Einführhülse (12) linear entlang der Längsachse (120) des Schafts (18) geführt ist.

7. Resektoskopvorrichtung (10) nach einem der vorhergehenden Ansprüche,
wobei der erste Schaftabschnitt (130) und die Einführhülse (12) gemeinsam eine Passung (150) bilden,
wobei insbesondere der erste Schaftabschnitt (130) eine Außenkontur (132) aufweist, die wenigstens abschnittsweise mit einem kreisrunden Außenquerschnitt (134) mit einem ersten Durchmesser (136) zusammenfällt, der derart bemessen ist, dass der erste Schaftabschnitt (130) in einen größten dem ovalen Querschnitt (110) der Einführhülse (12) einbeschreibbaren Kreis (116) eingepasst ist.

8. Resektoskopvorrichtung (10) nach einem der vorhergehenden Ansprüche,
wobei der erste Schaftabschnitt (130) proximal zum zweiten Schaftabschnitt (140) angeordnet ist, und / oder wobei der zweite Schaftabschnitt (140) zumindest teilweise ein distales Endstück (34) des Schafts (18) definiert.

9. Resektoskopvorrichtung (10) nach einem der vorhergehenden Ansprüche,
wobei der zweite Schaftabschnitt (140) eine Bilderfassungseinrichtung (36) umfasst, die einen Beobachtungsbereich (38) definiert.

10. Resektoskopvorrichtung (10) nach Anspruch 9,
wobei die Bilderfassungseinrichtung (36) zumindest eine Bilderfassungseinheit (42), insbesondere zur Stereobildgebung, umfasst, die durch zumindest eine Eingangsoptik (43) und/oder zumindest einen Bildsensor (45) zur Bilderzeugung ausgebildet ist, und
wobei die zumindest eine Eingangsoptik (43) insbesondere an einer distalen Endfläche (41) des Schafts (18) angeordnet ist.

11. Resektoskopvorrichtung (10) nach Anspruch 10,
wobei die Bilderfassungseinheit (42) eine Stereoeingangsoptik (160) mit zwei nebeneinander angeordneten Objektiven (162) aufweist, und wobei eine Summe der Durchmesser (164) der Objektive (162) größer ist als der Durchmesser (114) eines größten dem ovalen Querschnitt (110) der Einführhülse (12) einbeschreibbaren Kreises (116).

12. Resektoskopvorrichtung (10) nach einem der Ansprüche 9 bis **11,**
wobei die Bilderfassungseinrichtung (36) zumindest zwei Bilderfassungseinheiten (42) umfasst, die in unterschiedliche Blickrichtungen (44) gerichtet sind.

13. Resektoskopvorrichtung (10) nach einem der vorhergehenden Ansprüche, ferner umfassend:
ein Resektionswerkzeug (20),
wobei der Schaft (18) und das Resektionswerkzeug (20) unabhängig voneinander relativ zu der Einführhülse (12) entlang der Längsachse (120) des Schafts (18) bewegbar sind, und
wobei der Schaft (18) und das Resektionswerkzeug (20) dazu eingerichtet sind, nach einem Zuführen in eine Kavität (14) bei einem Abtragen von Gewebe innerhalb der Einführhülse (12) bewegt zu werden, wobei die Einführhülse (12) insbesondere dazu eingerichtet ist, bei dem Abtragen von Gewebe atraumatisch zu ruhen.

14. Resektoskopvorrichtung (10) nach Anspruch 13,
wobei das Resektionswerkzeug (20) zwei Trägerarme (26) und eine zwischen den Trägerarmen (26) angeordnete Werkzeugspitze (28), insbesondere eine HF-Resektionsschlinge (30), umfasst, und
wobei der Schaft (18) insbesondere zwischen den Trägerarmen (26) angeordnet ist.

15. Resektoskopvorrichtung (10) nach einem der vorhergehenden Ansprüche,
ferner umfassend eine proximale Bedienbaugruppe (50), die als Ganzes relativ zu der Einführhülse (12) bewegbar ist.

16. Resektoskop (60) mit einer Resektoskopvorrichtung (10) nach einem der vorhergehenden Ansprüche.
